# EUROPÄISCHE PATENTSCHRIFT

(11) **EP 3 684 909 B1**
(45) Veröffentlichungstag und Bekanntmachung des Hinweises auf die Patenterteilung: **07.04.2021**
(21) Anmeldenummer: 18826998.9
(22) Anmeldetag: 13.12.2018
(51) Int. Cl.: C12M 1/12, C12M 1/00

(54) **BIOREAKTOR UND DESSEN VERWENDUNG, VERFAHREN ZUR HERSTELLUNG EINER ORGANISCHEN NÄHRSTOFFLÖSUNG, ORGANISCHE NÄHRSTOFFLÖSUNG, SUBSTRATMATERIAL UND DESSEN VERWENDUNG ZUR KULTIVIERUNG VON PFLANZEN**
BIOREACTOR AND USE THEREOF, METHOD FOR PRODUCING AN ORGANIC NUTRIENT SOLUTION, ORGANIC NUTRIENT SOLUTION, SUBSTRATE MATERIAL AND USE THEREOF FOR CULTIVATING PLANTS
BIORÉACTEUR ET SON UTILISATION, PROCÉDÉ DE PRODUCTION D'UNE SOLUTION NUTRITIVE ORGANIQUE, SOLUTION NUTRITIVE ORGANIQUE, MATIÈRE DE SUBSTRAT ET SON UTILISATION POUR LA CULTURE DES VÉGÉTAUX

(30) Priorität: 22.12.2017 DE 102017131089
(43) Veröffentlichungstag der Anmeldung: 29.07.2020
(73) Patentinhaber: Jassen - Kunststoffzentrum GmbH - Apparatebau, Zuschnitte und Formung, 79585 Steinen (DE)
(72) Erfinder: ILLENBERGER, Bernhard, 79585 Steinen (DE); JASSEN, Hartmut, 79585 Steinen (DE)
(74) Vertreter: Mertzlufft-Paufler, Cornelius
(86) Internationale Anmeldenummer: PCT/EP2018/084682
(87) Internationale Veröffentlichungsnummer: WO 2019/121285

(56) Entgegenhaltungen:
- EP-A1- 0 389 958
- EP-A1- 0 486 748
- WO-A1-2010/003255
- WO-A1-2016/050893
- WO-A1-2017/076972
- DE-A1- 3 725 988
- DE-A1- 19 825 168
- ES-A1- 2 298 011
- US-A- 3 779 906

## Beschreibung

Die Erfindung betrifft einen Bioreaktor und dessen Verwendung zur Umwandlung von organischen Rest- und/oder Abfallstoffen in eine organische Nährstofflösung mit einem Anteil von mindestens 10% pflanzenverfügbarem mineralisiertem Stickstoff bezogen auf den Gesamtstickstoffgehalt der Nährstofflösung, mit einem Reaktionsbehälter, wobei der Reaktionsbehälter eine Zuleitung aufweist, über welche eine Suspension in den Reaktionsbehälter einleitbar ist, und wobei der Reaktionsbehälter eine Ableitung aufweist, über welche die Suspension aus dem Reaktionsbehälter ausleitbar ist.

Die Erfindung betrifft weiter ein Verfahren zur Herstellung einer organischen Nährstofflösung mit einem Anteil von mindestens 10% pflanzenverfügbarem Stickstoff.

Die Erfindung betrifft zudem auch ein Verfahren zur Herstellung eines Substratmaterials zur Kultivierung von Pflanzen.

Ferner betrifft die Erfindung eine organische Nährstofflösung. Insbesondere kann die organische Nährstofflösung ein organischer Pflanzendünger sein.

Die Erfindung betrifft außerdem ein Substratmaterial zur Kultivierung von Pflanzen mit einem Biofilm mit ammonifizierenden und/oder nitrifizierenden Bakterien. Zudem betrifft die Erfindung auch die Verwendung des Substratmaterials zur Kultivierung von Pflanzen.

Die Erfindung betrifft auch eine Verwendung ammonifizierender und/oder nitrifizierender Bakterien in Form eines Biofilms auf einem Trägerelement zur Umwandlung von organischen Rest-und/oder Abfallstoffen in eine organische Nährstofflösung mit einem Anteil von mindestens 10% pflanzenverfügbarem mineralisiertem Stickstoff bezogen auf den Gesamtstickstoffgehalt der Nährstofflösung.

Außerdem betrifft die Erfindung ein Set aus dem Bioreaktor und einem Impfmaterial zum Animpfen des Trägerelements und zur Ausbildung eines Biofilms mit ammonifizierenden und/oder nitrifizierenden Bakterien.

Weiter betrifft die Erfindung ein Set aus einem Trägerelement und einem Impfmaterial zum Animpfen des Trägerelements und zur Ausbildung eines Biofilms mit ammonifizierenden und/oder nitrifizierenden Bakterien.

Die Landwirtschaft hat schon immer Nährstoffe für die landwirtschaftliche Produktion von Pflanzen aus der Nutzung und Verwertung von pflanzlichen und tierischen Rest- und Abfallstoffen gewonnen. Der ökologische Landbau hat sich die Regel auferlegt, dass Stickstoff als wichtiger Pflanzennährstoff nur in organischer Form als Wirtschafts-/Handelsdünger wie z. B. Gülle, Komposte oder Hornmehl ausgebracht werden darf. Durch die Ausweitung des Marktes für ökologisch erzeugte Produkte nimmt die Spezialisierung der Betriebe zu und Gemüseanbauern und andere Betriebe ohne Tierhaltung sind gezwungen, aufgrund des Verkaufs und Exports der Biomasse und des Fehlens von Wirtschaftsdünger organischen Handelsdünger zuzukaufen und einzusetzen. Gerade beim stärker nachgefragten Einsatz von organischen Düngern besteht ein beträchtlicher Anteil von nicht mineralisiertem, für die Pflanzen nicht sofort aufnehmbarem Stickstoff.

Als pflanzenverfügbaren Stickstoff, also Stickstoff der direkt von Pflanzen aufgenommen werden kann, werden grundsätzlich anorganische Stickstoffverbindungen verstanden. Im Gegensatz dazu können organische Stickstoffverbindungen von Pflanzen nicht direkt aufgenommen werden. Der Begriff "pflanzenverfügbarer Stickstoff" bezieht sich in der Regel auf Stickstoff, der in Form von Ammonium (NH₄⁺) und Nitrat (NO₃) vorliegt.

Hydroponische, erdlose bzw. erdarme Kultivierungssysteme für Pflanzen nutzen derzeit mineralischen Dünger bzw. mineralisch organischen Dünger (Dünger dem zum organischen Material mineralisierte Stickstoffverbindungen aus industrieller Produktion beigemischt sind). Rein organisches Düngermaterial kann aus zwei Gründen nicht verwendet werden. Zum einen ist die bakterielle Boden-Pflanze-Interaktion in solchen Kultivierungssystemen sehr stark eingeschränkt bzw. entfällt bei erdlosen Ansätzen. Zudem können sofort pflanzenverfügbare mineralisierte Nährstoffe im Boden nicht in ausreichender Menge durch einen von äußeren Einflüssen abhängigen bakteriellen Prozess, wie der Nitrifizierung, zur Verfügung gestellt werden. Darüber hinaus ist mit organischen Düngern eine kontrollierte, stetige und sofortige Stickstofffreisetzung, die die Pflanze insbesondere in hydroponischen Pflanzsystemen benötigt, nicht zu erreichen.

Weiter als problematisch anzusehen ist, dass die Umsetzung von organischem Düngermaterial, wie Kompostabfällen und dergleichen, und die damit verbundene Freisetzung von mineralisiertem Stickstoff von einer Vielzahl schwer kontrollierbarer Umweltfaktoren wie Bodenfeuchte, Bodentemperatur sowie unterschiedlichen Konzentrationen organischer Verbindungen im Boden abhängig ist. Dieser Mangel an Kontrolle führt bei der landwirtschaftlichen Anwendung zu nicht zeitgerechter Zurverfügungstellung und Überschüssen/Defiziten in der Stickstoffversorgung sowie zu wirtschaftlich und umweltgefährdenden Gegenprozessen wie beispielsweise zur Denitrifikation.

Insbesondere für erdlose (bzw. erdarme) hydroponische/aeroponische Kultivierungssysteme ist es essentiell, dass die Pflanzen mit einer optimal eingestellten Nährstofflösung versorgt werden, da durch den fehlenden bzw. reduzierten Erdkörper keine Pufferfunktion des Substrates im Hinblick auf Nährstoff- und Wasserversorgung gegeben ist. Die Nutzung von herkömmlichem, organischem Düngermaterial ist bei diesen Systemen ungeeignet, da der Stickstoff darin nicht oder nur unzureichend pflanzenverfügbar ist. Darüber hinaus lässt sich mit organischem Düngermaterial aufgrund weiterhin stattfindender Ab- bzw. Umbauprozesse keine definierte und stabile Nährstoffkonzentration in der Nährlösung einstellen.

Neben dem grundsätzlichen Nachteil des biologischen Landbaus, der auf den Einsatz von industriell gewonnenem mineralischem Stickstoffdünger verzichtet, der ein wesentliches Mittel der Ertragssteigerung wäre, ist weiterhin als problematisch anzusehen, dass der Bedarf an sofort pflanzenverfügbarem Stickstoff insbesondere im letzten Drittel der Wachstumsphase von Nutzpflanzen durch den Einsatz von bisher erhältlichen organischen Wirtschaftsdüngern sowie organischen Düngemitteln aus Rest- und Abfallstoffen nicht rechtzeitig gedeckt werden kann.

Die Druckschrift EP 0 486 748 A1 offenbart einen Biofilmreaktor zur Behandlung von organisch belasteten Abwasser. In den Reaktor werden Biofilm-Träger-Teilchen eingebracht, an denen sich Bakterien Teilchen anlagern können.

Das Dokument WO 2016/050893 A1 offenbart die Verwendung von Biofilmträgern in anaeroben Abbauprozessen, wobei ein Biofilm aus Methan produzierenden Bakterien vorgesehen ist.

Die Druckschrift WO 2010/003255 A1 offenbart eine Einrichtung, mit der Wasser mit Sauerstoff angereichert werden kann. Das mit Sauerstoff angereicherte Wasser wird sodann in eine Pflanzenkultur eingebracht.

Es besteht daher die Aufgabe einen Bioreaktor, ein Verfahren und ein Substratmaterial zu schaffen, womit es möglich ist, eine rein organische Nährstofflösung aus einem organischen Rest- und/oder Abfallstoff herzustellen, wobei ein Anteil an pflanzenverfügbarem Stickstoff der organischen Nährstofflösung höher als bei bisher erhältlichen organischen Düngern liegt.

Die Aufgabe wird erfindungsgemäß gelöst durch die Merkmale von Anspruch 1. Insbesondere wird somit zur Lösung der genannten Aufgabe bei einem Bioreaktor der eingangs beschriebenen Art vorgeschlagen, dass der Bioreaktor eine Belüftungseinrichtung aufweist, mittels welcher Sauerstoff in den Reaktionsbehälter und die darin enthaltene Suspension einleitbar ist, dass in einem Aufnahmeraum des Reaktionsbehälters wenigstens ein Trägerelement mit einer Ansiedlungsfläche zur Ausbildung eines Biofilms aus Mikroorganismen angeordnet ist, wobei das wenigstens eine Trägerelement mit der eingeleiteten Suspension und dem eingeleiteten Sauerstoff umspülbar und/oder durchspülbar ist, und dass ein Oberfläche-zu-Volumen-Verhältnis des Trägerelements größer als ein Oberfläche-zu-Volumen-Verhältnis des Aufnahmeraums ausgestaltet ist. Anhand des Bioreaktors ist es so möglich, einen Biofilm an gewünschter Stelle auf der Ansiedlungsfläche innerhalb eines Aufnahmeraums auszubilden. An der Ansiedlungsfläche können so ideale Wachstumsbedingungen für ammonifizierende und/oder nitrifizierende Bakterien erzeugt werden. Durch den Bioreaktor können somit die gewünschten Umsatzreaktionen der Mikroorganismen besser unterstützt werden, wodurch organische Nährstofflösungen mit einem größeren Anteil an pflanzenverfügbarem Stickstoff herstellbar sind. Besonders bevorzugt kann es dabei sein, wenn das Oberfläche-zu-Volumen-Verhältnis des Trägerelements mindestens achtmal größer als das Oberfläche-zu-Volumen-Verhältnis des Aufnahmeraums ausgestaltet ist. Die Belüftungseinheit kann beispielsweise dazu eingerichtet sein, den Sauerstoff in Form von Luft einzuleiten.

Gemäß einer vorteilhaften Weiterbildung kann das wenigstens eine Trägerelement aus einem Material oder einer Kombination von zwei oder mehreren Materialien ausgewählt aus der Gruppe aus Kunststoff, insbesondere aus lebensmittelneutralem Kunststoff, Mineral, insbesondere aus Zeolith, und/oder einer Gummi-Kunststoff-Mischung hergestellt sein. Besonders zweckmäßig kann es dabei sein, wenn es sich bei dem Kunststoff um Polypropylen und/oder Polyethylen und/oder Gummi handelt, da sich die Mikroorganismen an diesen Kunststoffen besonders gut ansiedeln lassen und einen Biofilm ausbilden. Weiter kann es zweckmäßig sein, wenn es sich um eine Polyethylen-Gummi-Mischung handelt. Es ist auch denkbar, dass eine Mineral-Kunststoffmischung, insbesondere eine Zeolith-Kunststoffmischung vorgesehen ist.

Besonders vorteilhaft kann es weiter sein, wenn die Ansiedlungsfläche des Trägerelements zumindest teilweise in Poren und/oder Hohlräumen ausgebildet ist. Dies hat den Vorteil, dass das Trägerelement eine möglichst große, besiedelbare Oberfläche bei relativ geringen Außenabmessungen des Trägerelements aufweist. Vorzugsweise können die Poren und/oder die Zugänge zu den Hohlräumen einen Durchmesser von 10 µm bis 100 µm aufweisen, so dass die Poren und/oder Zugänge ein Durchdringen und/oder Eindringen von Flüssigkeit und Gas und ein Eindringen und Festsetzen von Mikroorganismen erlauben. Besonders zweckmäßig kann es sein, wenn die Poren zumindest teilweise oder größtenteils durchgängig ausgebildet sind, das Material also insbesondere offenporig ausgestaltet ist. Somit kann eine besonders gute Durchspülung mit Suspension erreicht werden.

Alterativ oder ergänzend kann es gemäß einer weiteren vorteilhaften Ausgestaltung vorgesehen sein, dass das wenigstens eine Trägerelement aus dem Reaktionsbehälter herausnehmbar, insbesondere werkzeuglos herausnehmbar, ausgestaltet ist. Insbesondere kann das Trägerelement als austauschbares Modul ausgestaltet sein. Dies hat den Vorteil, dass ein schnelles und einfaches Herausnehmen und Austauschen des Trägerelements aus dem Reaktionsbehälter möglich ist. Besonders zweckmäßig kann es dabei weiter sein, wenn im Aufnahmeraum eine Führungseinrichtung angeordnet ist, mittels welcher das Trägerelement in geführter Art in den Aufnahmeraum einführbar ist, insbesondere einschiebbar oder einsteckbar ist. Eine Position des Trägerelements innerhalb des Aufnahmeraums kann somit durch die Führungseinrichtung definiert sein.

Um das Einleiten von Sauerstoff in den Reaktionsbehälter zu erleichtern und um anaerobe Umsetzungen noch besser zu vermeiden, kann es vorgesehen sein, dass mittels der Belüftungseinrichtung über eine Gaszuführleitung Sauerstoff in den Reaktionsbehälter und/oder in das wenigstens eine Trägerelement einleitbar ist. Dabei kann es vorteilhaft sein, wenn die Belüftungseinrichtung zumindest einen Kompressor oder einen Gasanschluss aufweist, um die Einleitung besser regulieren zu können. Vorzugsweise kann die Gaszuführleitung von der Zuleitung und der Ableitung unabhängig sein, so dass Sauerstoff und Suspension unabhängig voneinander einleitbar sind. Der Sauerstoff kann dabei beispielsweise auch in Form von Luft eingeleitet werden. Dies hat den Vorteil, dass insbesondere kein reiner Sauerstoff, sondern Umluft eingeleitet wird, die in unbegrenzter Menge zur Verfügung steht.

Alternativ oder ergänzend kann an einem Boden des Reaktionsbehälters die Belüftungseinrichtung, insbesondere eine oder die bereits zuvor genannte als Belüftungsplatte ausgebildete Belüftungseinrichtung, mit einer Vielzahl an Belüftungsöffnungen ausgebildet sein. Insbesondere kann mittels der Belüftungseinrichtung eine gleichmäßige Gasblasendichte innerhalb des Reaktionsbehälters, vorzugsweise innerhalb des Aufnahmeraums, erzeugbar sein, so dass eine besonders gleichmäßige Belüftung innerhalb des Reaktionsbehälters möglich ist. Vorzugsweise können die Belüftungsöffnungen dabei gleiche Durchmesser aufweisen und/oder in gleichen Abständen zueinander angeordnet sein. Weiter bevorzugt kann die Belüftungseinrichtung kegelförmig ausgestaltet sein, so dass besser verhindert werden kann, dass sich die Belüftungsöffnungen durch absinkende feste Teilchen der Suspension zusetzen.

Um eine besonders gute Umwälzung der Suspension innerhalb des Aufnahmeraums zu erreichen, kann der Bioreaktor eine Pumpvorrichtung aufweisen. Die Pumpvorrichtung kann beispielsweise als Kreiselpumpe oder Umwälzpumpe ausgestaltet sein. Weiter kann es vorteilhaft sein, wenn mittels der Pumpvorrichtung die Suspension durch die Zuleitung in den Reaktionsbehälter und über die Ableitung aus dem Reaktionsbehälter pumpbar ist. Um unterschiedliche Durchflussmengen durch den Reaktionsbehälter und/oder die Zu-und Ableitung einstellen zu können, kann eine Pumpleistung der Pumpvorrichtung veränderbar, insbesondere also manuell einstellbar und/oder programmierbar, sein. Vorzugsweise kann mittels der Pumpvorrichtung eine Suspensionsströmungsrichtung innerhalb des Reaktionsbehälters erzeugbar sein, die wenigstens teilweise entgegen und/oder wenigstens teilweise in einer Sauerstoffströmungsrichtung verläuft. Dadurch ist eine besonders gute Durchmischung der Suspension mit Sauerstoff erreichbar. Um mittels des Bioreaktors die Durchführung eines Herstellungsverfahrens aus mehreren Schritten automatisieren zu können, kann vorzugsweise die Pumpvorrichtung derart programmierbar sein, dass ein Pumpprogramm mit mehreren Teilschritten automatisiert einstellbar ist.

Um ideale Wachstums- und/oder Umsetzungsbedingungen für die Mikroorganismen zu schaffen, kann der Bioreaktor eine Heizvorrichtung aufweisen, mit der der Aufnahmeraum des Reaktionsbehälters und/oder die im Aufnahmeraum des Reaktionsbehälters enthaltene Suspension auf eine einstellbare Temperatur aufheizbar ist. Insbesondere kann eine Temperatur von 20°C bis 34°C, vorzugsweise eine Temperatur von 22°C bis 32°C, vorgesehen sein.

Damit das Trägerelement einfach aus dem Bioreaktor entnehmbar ist, kann gemäß einer vorteilhaften Weiterbildung vorgesehen sein, dass der Reaktionsbehälter eine Öffnung zur Befüllung des Aufnahmeraums des Reaktionsbehälters aufweist, und dass der Bioreaktor eine Verschlusseinheit aufweist, mit welcher Verschlusseinheit die Öffnung des Reaktionsbehälters verschließbar ist. Vorzugsweise kann die Öffnung des Reaktionsbehälters mittels der Verschlusseinheit flüssigkeitsdicht und/oder druckbeständig verschließbar sein.

Um störende äußere Einflüsse auf die Umwandlung des organischen Rest- und/oder Abfallstoffes in die organische Nährstofflösung zu vermeiden, kann eine Wandung des Reaktionsbehälters und eine oder die bereits zuvor genannte Verschlusseinheit lichtundurchlässig ausgebildet sein. Somit kann die Umwandlungsrate verbessert werden.

Es kann weiter zweckmäßig sein, wenn die Ansiedlungsfläche hydrophob ist.

Um den Mikroorganismen das Festhalten auf der Ansiedlungsfläche zu erleichtern, kann die Ansiedlungsfläche eine höhere Rauigkeit als eine Innenseite einer Reaktionsbehälterwandung aufweisen.

Um eine möglichst große Gesamtansiedlungsfläche zur Ansiedlung der Mikroorganismen bereitzustellen, kann der Bioreaktor mehrere Trägerelemente aufweisen. Vorzugsweise können die Trägerelemente dabei relativ zueinander beweglich ausgestaltet und/oder angeordnet sein. Vorzugsweise sind die Trägerelemente lose innerhalb des Aufnahmeraums angeordnet. Durch eine Umwälzung der Suspension können somit auch die Trägerelemente durch die entstehende Strömung ebenfalls umgewälzt und/oder bewegt werden. Somit kann ein Gas- und Suspensionsaustausch an den Trägerelementen verbessert werden.

Gemäß einer besonders bevorzugten Weiterbildung kann als Trägerelemente eine Vielzahl von Spänen, insbesondere eine Vielzahl von Fließspänen aus Kunststoff, vorgesehen sein, wobei die Späne ungeordnet innerhalb des Reaktionsbehälters angeordnet sein können. Insbesondere kann durch die ungeordnete Anordnung ein Verknäulen der Späne entstehen. Somit ist es möglich, dass die Ansiedlungsflächen der Späne nicht aneinander anhaften und/oder nicht ausreichend mit Sauerstoff umspülbar sind, so dass keine ungewollten Denitrifizierungsprozesse ablaufen können. Besonders bevorzugt kann es dabei sein, wenn die Späne eine Form oder eine Kombination von mehreren Formen ausgewählt aus der Gruppe aus spiralförmige und/oder mäanderförmige und/oder wellige Späne aufweisen. Somit kann besonders gut verhindert werden, dass Ansiedlungsflächen von Spänen aneinander anhaften oder anliegen.

Vorzugsweise können die Späne jeweils eine Länge von 2 cm bis 10 cm und/oder eine Breite von 0,5 cm bis 1,5 cm und/oder eine Dicke von 50 µm bis 500 µm aufweisen.

Alternativ oder ergänzend kann als Trägerelement wenigstens ein poröser Schlauch vorgesehen sein. Insbesondere kann der Schlauch quer oder parallel zu einer Strömungsrichtung des in den Reaktionsbehälter eingeleiteten Sauerstoffs angeordnet sein und/oder quer oder parallel zu einer Strömungsrichtung der in den Reaktionsbehälter eingeleiteten Suspension angeordnet sein. Somit ist eine besonders gute Umspülung des Schlauches möglich.

Gemäß einer vorteilhaften Weiterbildung kann es vorgesehen sein, dass über eine Gaszuführleitung Sauerstoff durch einen oder den zuvor genannten Schlauch einleitbar ist. Insbesondere kann diese Gaszuführleitung als eine Bypassgasleitung ausgestaltet sein, die von einer Hauptgasleitung, vorzugsweise zu einer oder der zuvor genannten Belüftungseinrichtung führenden Hauptgasleitung, abzweigt. Somit ist eine besonders gute Versorgung mit Sauerstoff der an einer Innenseite der Schlauchwandung angesiedelten Mikroorganismen möglich. Vorzugsweise weist der Bioreaktor mehrere als Schläuche ausgebildete Trägerelemente auf. Es kann weiter vorgesehen sein, dass der Schlauch in eine innerhalb des Aufnahmeraums verlaufende Leitung integriert ist. Diese Leitung kann mit der Zuleitung und/oder der Ableitung verbunden sein. Vorzugsweise kann vor und/oder nach dem Schlauch ein Absperrventil in die Leitung innerhalb des Aufnahmeraums eingesetzt sein. Somit lässt sich auf einfache Weise durch Schließen eines in Strömungsrichtung nach dem Schlauch angeordneten Absperrventils ein Schlauchinnendruck erhöhen, womit die Strömung der Suspension zumindest kurzfristig durch die Poren in der Schlauchwandung leitbar ist oder durch Schließen eines in Strömungsrichtung vor dem Schlauch angeordneten Absperrventils ein Einleiten von Suspension in den Schlauch verhinderbar ist.

Gemäß einer weiteren vorteilhaften Weiterbildung kann es vorgesehen sein, dass das Trägerelement als Zeolith-Granulat ausgestaltet ist. Insbesondere kann das Zeolith-Granulat eine Körnung von 0,6 mm bis 1,0 mm aufweisen. Vorzugsweise kann/können das Zeolith-Granulat und/oder ein anderes Trägermaterial in einer innerhalb des Reaktionsbehälters angeordneten vorzugsweise als Textilbeutel ausgebildeten Sammeleinheit angeordnet sein. Somit kann verhindert werden, dass das Trägermaterial in schlecht belüftbare und/oder schlecht durchmischbare Bereiche innerhalb des Aufnahmeraums geschwemmt wird.

Alternativ oder ergänzend kann an einem oder dem Boden einer oder der bereits zuvor genannten Sammeleinheit eine weitere Belüftungseinrichtung angeordnet sein, über welche Sauerstoff in das Zeolith-Granulat und/oder ein anderes Trägerelement einleitbar ist. Vorzugsweise kann die weitere Belüftungseinrichtung mit einer von einer Hauptgasleitung abgezweigten Bypassgasleitung verbunden sein. Durch die weitere Belüftungseinrichtung ist eine besonders gute Umspülung des Zeolith-Granulats und/oder eines anderen Trägerelements mit Sauerstoff möglich.

Um ein Absetzen von Feststoffen der Suspension am Boden des Aufnahmeraums besser vermeiden zu können, kann die Zuleitung oberhalb und/oder auf gleicher Höhe mit der Ableitung in den Reaktionsbehälter münden. Somit können absinkende Teilchen vorzugsweise weiter oberhalb in den Aufnahmeraum eingeleitet und weiter unten aus dem Aufnahmeraum ausgeleitet werden.

Alternativ oder ergänzend kann der Bioreaktor mehrere Zuleitungen und/oder mehrere Ableitungen aufweisen. Vorzugsweise wobei zumindest teilweise durch eine einstellbare Suspensionsströmungsrichtung definiert sein kann, ob eine Leitung des Bioreaktors während eines Gebrauchs des Bioreaktors eine Aufgabe einer Zuleitung oder einer Ableitung hat. Dies ermöglicht es, mindestens zwei unterschiedliche Strömungsrichtungen innerhalb des Reaktionsbehälters, vorzugsweise gleichzeitig, einzurichten, wobei bestehende Leitungen abhängig von der Strömungsrichtung als Zu- oder Ableitung nutzbar sind. So können beispielsweise wenigstens zwei Ableitungen in Bodennähe aus dem Reaktionsbehälter führen, um abgesetzte Teilchen und Suspension noch effizienter ableiten zu können.

Damit innerhalb des Aufnahmeraums des Reaktionsbehälters eine besonders gute Umwälzung der Suspension erreichbar ist, können mittels einer oder der hierin zuvor bereits genannten Pumpvorrichtung wenigstens zwei Suspensionsströmungsrichtungen innerhalb des Reaktionsbehälters und/oder innerhalb der Leitungen des Bioreaktors einstellbar sein. Zur Erreichung einer besonders guten Umwälzung können alternativ oder ergänzend die Zuleitung und die Ableitung durch eine oder die hierin zuvor bereits genannte Pumpvorrichtung voneinander getrennt sein. Insbesondere kann der Bioreaktor einen die Zuleitung, die Ableitung, die Pumpvorrichtung und den Reaktionsbehälter umfassenden Suspensionskreislauf aufweisen, so dass die Suspension mehrfach an dem Biofilm innerhalb des Reaktionsbehälters vorbeiführbar ist.

Zur einfacheren Regelung der Suspensionsströmung kann in der Zuleitung zwischen dem Reaktionsbehälter und einer oder der Pumpvorrichtung ein Absperrventil angeordnet sein und/oder in der Ableitung zwischen dem Reaktionsbehälter und einer oder der Pumpvorrichtung ein Absperrventil angeordnet sein.

Gemäß einer vorteilhaften Weiterbildung des Bioreaktors kann von der Zuleitung, insbesondere nach einer oder der Pumpvorrichtung und/oder vor einem oder dem Absperrventil, eine Bypasszuleitung abzweigen, die in den Reaktionsbehälter mündet. Somit kann eine Suspension zusätzlich auch über die Bypasszuleitung in den Reaktionsbehälter eingeleitet werden. Vorzugsweise kann die Bypasszuleitung ein Absperrventil aufweisen. Weiter bevorzugt kann es vorgesehen sein, dass die Bypasszuleitung bei einer Änderung, insbesondere einer Umkehr, der Suspensionsströmungsrichtung durch die Pumpvorrichtung als Ableitung verwendbar ist. Somit kann die Variabilität der Strömungswege erhöht werden, was zu einer noch besseren Umwälzung der Suspension im Reaktionsbehälter führt.

Es kann besonders zweckmäßig sein, wenn auf der Ansiedlungsfläche ein Biofilm angesiedelt ist, der ammonifizierende und/oder nitrifizierende Bakterien aufweist. Dabei kann es beispielsweise vorgesehen sein, dass ein Anteil ammonifizierender und/oder nitrifizierender Bakterien am Biofilm mindestens 2%, vorzugsweise mindestens 4%, vorzugsweise mindestens 6%, vorzugsweise mindestens 10%, vorzugsweise mindestens 15%, vorzugsweise mindestens 20%, vorzugsweise mindestens 25%, vorzugsweise mindestens 30%, vorzugsweise mindestens 40%, vorzugsweise mindestens 50%, vorzugsweise mindestens 60%, vorzugsweise mindestens 70%, vorzugsweise mindestens 80%, vorzugsweise mindestens 90%, vorzugsweise annähernd 100%, ausmacht.

Um eine möglichst große Oberfläche zur Ansiedlung der Mikroorganismen zu erzeugen, kann das wenigstens eine Trägerelement mehrere Ansiedlungsflächen aufweisen, wobei die Ansiedlungsflächen gekrümmt ausgestaltet sein können, so dass eine Anhaftung und/oder ein Anliegen von Ansiedlungsflächen eines Trägerelements aneinander und/oder eine Anhaftung und/oder ein Anliegen der Ansiedlungsflächen unterschiedlicher Trägerelemente aneinander vermeidbar ist.

Die oben genannte Aufgabe wird außerdem erfindungsgemäß gelöst durch die Merkmale des unabhängigen Verfahrensanspruchs zur Herstellung einer organischen Nährstofflösung. Insbesondere wird somit zur Lösung der genannten Aufgabe ein Verfahren zur Herstellung einer organischen Nährstofflösung vorgeschlagen, wobei die organische Nährstofflösung einen Anteil von mindestens 10%, insbesondere mindestens 25%, vorzugsweise mindestens 50%, weiter bevorzugt mindestens 75%, pflanzenverfügbarem, mineralisiertem Stickstoff bezogen auf den Gesamtstickstoffgehalt der organischen Nährstofflösung aufweist. Vorzugsweise wobei ein Nitratanteil am pflanzenverfügbaren, mineralisierten Stickstoff höher als ein Ammoniumanteil ist. Besonders bevorzugt kann dabei sein, dass in der organischen Nährlösung ein NO3:NH4⁺-Verhältnis von mindestens 2:1, insbesondere von mindestens 3:1, insbesondere von mindestens 10:1, vorzugsweise von mindestens 25:1, weiter bevorzugt von mindestens 50:1, vorliegt. Das Verfahren, umfasst dabei die Schritte:
- in einem Animpfschritt Animpfen eines Trägerelements, vorzugsweise eines Trägerelements des Bioreaktors wie er hierin beschrieben und beansprucht ist, mit einem Impfmaterial das ammonifizierende und/oder nitrifizierende Bakterien enthält,
- Ausbilden eines Biofilms mit ammonifizierenden und/oder nitrifizierenden Bakterien auf dem Trägerelement,
- in einem Inkubationsschritt Inkubation eines organischen Rest- und/oder Abfallstoffes mit dem Biofilm, insbesondere in einem Reaktionsbehälter, wobei die ammonifizierenden und/oder die nitrifizierenden Bakterien organisch gebundenen Stickstoff des Rest- und/oder Abfallstoffes in mineralisierten Stickstoff umwandeln.

Durch das Verfahren ist es erstmals möglich, aus organischen Rest- und/oder Abfallstoffen eine als Pflanzendünger verwendbare organische Nährstofflösung herzustellen, bei welcher der Anteil an pflanzenverfügbarem Stickstoff aufgrund des speziellen Verfahrens drastisch erhöht wurde. Gemäß einer bevorzugten Ausgestaltung kann damit bei dem pflanzenverfügbaren Stickstoff zudem eine deutliche Verschiebung des Verhältnisses des Nitrat- zu Ammoniumanteils hin zum Nitrat erreicht werden. Insbesondere in erdlosen, hydroponischen Systemen sind Pflanzen vor allem auf Nitrat angewiesen, da dieses besser aufgenommen werden kann. Darüber hinaus ist es zudem möglich, die oftmals bei organischen Rest-und/oder Abfallstoffen auftretende Geruchsbelastung, welche insbesondere durch Ammonium entstehen kann, deutlich zu reduzieren bis hin zu einer kompletten Geruchsneutralisation bei der fertigen organischen Nährstofflösung.

Die oben genannte Aufgabe wird außerdem erfindungsgemäß gelöst durch die Merkmale des unabhängigen Verfahrensanspruchs zur Herstellung eines Substratmaterials zur Kultivierung von Pflanzen. Insbesondere wird somit zur Lösung der genannten Aufgabe ein Verfahren zur Herstellung eines Substratmaterials zur Kultivierung von Pflanzen vorgeschlagen, umfassend die Schritte:
- in einem Animpfschritt Animpfen eines Trägerelements, vorzugsweise eines Trägerelements des Bioreaktors nach einem der vorstehenden Ansprüche, mit einem Impfmaterial das ammonifizierende und/oder nitrifizierende Bakterien enthält,
- Ausbilden eines Biofilms mit ammonifizierenden und/oder nitrifizierenden Bakterien auf dem Trägerelement.

Durch das zuvor beschriebene Verfahren ist es somit möglich, Trägerelemente mit ammonifizierenden und/oder nitrifizierenden Bakterien anzuimpfen und daraus einen Biofilm auf dem Trägerelement wachsen zu lassen. Das Trägerelement kann dann beispielsweise in einem Biorekator, wie hierin beschrieben und beansprucht, oder als Substratmaterial, wie hierin beschrieben und beansprucht, eingesetzt werden.

Nachfolgende vorteilhafte Weiterbildungen beziehen sich auf beide zuvor beschriebenen Verfahren.

Um eine besonders gute Animpfung und/oder Umwandlung erreichen zu können, kann das Impfmaterial und/oder der organische Rest-und/oder Abfallstoff in flüssiger Form verwendet werden. Vorzugsweise kann das Impfmaterial und/oder der organischen Rest- und/oder Abfallstoff in einer Suspensionsform verwendet werden. Somit ist eine besonders gute Benetzung des Trägerelements mit dem Impfstoff und/oder dem organischen Rest-und/oder Abfallstoff möglich. Weiter ist eine besonders einfach durchführbare und effektive Umwälzung des organischen Rest-und/oder Abfallstoffes möglich. Hierzu kann beispielsweise eine Vermischung eines als Feststoff vorliegenden Impfmaterials und/oder organischen Rest- und/oder Abfallstoffs mit Wasser vorgesehen sein, um eine Suspension herzustellen.

Es kann vorteilhaft sein, wenn das Impfmaterial oder eine Kombination aus einem oder mehreren Impfmaterialen ausgewählt aus der Gruppe aus Kompost, insbesondere Rindenkompost, Wurmexkremente, insbesondere Regenwurmexkremente, Erde, insbesondere Felderde, verwendet wird. Diese Impfmaterialien enthalten neben ammonifizierenden und/oder nitrifizierenden Bakterien zusätzlich Schleim- und/oder Eiweißstoffe, die die Ausbildung des Biofilms auf dem Trägerelement beschleunigen und den Biofilm stabilisieren. Grundsätzlich sind Exkremente von im Boden lebenden Würmern geeignet, da diese reich an ammonifizierenden und/oder nitrifizierenden Bakterien sind.

Gemäß einer besonders vorteilhaften Ausgestaltung kann ein Anteil an organischem Material des organischen Rest- und/oder Abfallstoffes von 5% bis 60% betragen.

Alternativ oder ergänzend kann ein Kohlenstoff/Stickstoff-Verhältnis des organischen Rest- und/oder Abfallstoffes 11 oder weniger betragen. Bei Werten über 11 verschlechtern sich die Umwandlungsraten.

Weiter kann es vorteilhaft sein, wenn ein Gesamtstickstoffanteil bezogen auf das Gesamtgewicht des organischen Rest- und/oder Abfallstoffes wenigstens 0,3% beträgt.

Es kann weiter vorgesehen sein, dass ein Anteil an nitratgebundenem Stickstoff bezogen auf den Gesamtgehalt des pflanzenverfügbaren Stickstoffs des organischen Rest- und/oder Abfallstoffes geringer als ein Anteil an ammmoniumgebundenem Stickstoff ist. Durch das Verfahren kann dieses Verhältnis verschoben werden, so dass mehrheitlich Nitrat anstelle von Ammonium vorliegt.

Um ideale Wachstumsbedingungen für die ammonifizierenden und/oder nitrifizierenden Bakterien zu schaffen, können einzelne oder alle Verfahrensschritte bei einer Temperatur, insbesondere bei einer konstanten Temperatur, von 20°C bis 34°C, vorzugsweise von 22°C bis 32°C, durchgeführt werden.

Um unerwünschte Denitrifizierungsprozesse durch anaerobe denitrifizierende Bakterien zu verhindern und um die aeroben ammonifizierenden und/oder nitrifizierenden Bakterien ausreichend mit Sauerstoff zu versorgen, kann während der Durchführung einzelner oder aller Schritte Sauerstoff in den Reaktionsbehälter und/oder in das Trägerelement eingeleitet werden.

Um besser verhindern zu können, dass sich während des Animpfschrittes anaerobe Bakterien ansiedeln, kann das Impfmaterial während des Animpfschritts umgewälzt werden. Insbesondere indem das Impfmaterial mehrmals und/oder in unterschiedlichen Strömungsrichtungen durch einen, vorzugsweise geschlossenen, Kreislauf gepumpt wird, in welchem Kreislauf das Trägerelement angeordnet ist. Insbesondere kann durch die Umwälzung am Trägerelement festgesetztes Impfmaterial und Teile des Biofilms abgerissen werden und sich an anderer Stelle erneut anhaften. Dieses Abreißen und erneutes Anhaften fördert das Bakterienwachstum.

Alternativ oder ergänzend kann der organische Rest- und/oder Abfallstoff während des Inkubationsschritts umgewälzt werden, um ein Absetzen von Feststoffen und das Auftreten von anaeroben Abbauprozessen besser zu verhindern. Insbesondere kann der organische Rest- und/oder Abfallstoff mehrmals und/oder in unterschiedlichen Strömungsrichtungen durch einen, vorzugsweise geschlossenen, Kreislauf gepumpt werden, in welchem Kreislauf das Trägerelement angeordnet ist.

Um die Umwandlungseffizienz der Bakterien weiter erhöhen zu können, kann der Animpfschritt in eine erste Phase mit kontinuierlicher Belüftung und/oder Umwälzung und eine zweite Phase mit diskontinuierlicher Belüftung und/oder Umwälzung unterteilt sein. Vorzugsweise können dabei während der zweiten Phase Belüftungspausen und/oder Umwälzungspausen, insbesondere von 30 bis 50 Minuten pro Stunde, durchgeführt werden.

Es kann weiter zweckmäßig sein, wenn der Animpfschritt und der Inkubationsschritt in unterschiedlichen Reaktionsbehältern durchgeführt werden. Vorzugsweise kann der während des Animpfschritts verwendete Reaktionsbehälter ein geringeres Volumen als der während des Inkubationsschritts verwendete Reaktionsbehälter aufweisen.

Es kann weiter vorgesehen sein, dass der Inkubationsschritt einen Ammonifizierungsschritt und/oder einen Nitrifizierungsschritt aufweist, wobei während des Ammonifizierungsschritts durch die ammonifizierenden Bakterien des Biofilms aus dem organischen Rest- und/oder Abfallstoff organisch gebundener Stickstoff in Ammonium umgewandelt wird und/oder wobei während des Nitrifizierungsschritts durch die nitrifizierenden Bakterien des Biofilms Ammonium in Nitrat umgewandelt wird. Je nachdem welcher organische Rest- und/oder Abfallstoff eingesetzt wird und welche anfänglichen Anteile an pflanzenverfügbarem Stickstoff er aufweist, kann mitunter nur ein Nitrifizierungsschritt vorgesehen sein. Insbesondere kann der Inkubationsschritt solange durchgeführt werden, bis in der organischen Nährlösung mehr Nitrat als Ammonium enthalten ist, vorzugsweise bis ein N03:NH4+-Verhältnis von mindestens 2:1, insbesondere von mindestens 3:1, vorzugsweise von mindestens 10:1, vorzugsweise von mindestens 25:1, weiter bevorzugt von mindestens 50:1, vorliegt. Um festzustellen, welcher Anteil pflanzenverfügbarer Stickstoff vorliegt, kann vorzugsweise in regelmäßigen Abständen oder kontinuierlich ein Messschritt durchgeführt werden. Dabei kann auf für den Fachmann bekannte Messmethoden zur Bestimmung einer Nitrat- und/oder Ammoniumkonzentration und/oder einer Gesamtstickstoffkonzentration zurückgegriffen werden.

Gemäß einer besonders vorteilhaften Weiterbildung kann als Trägerelement wenigstens ein poröser Schlauch, insbesondere ein poröser Gummi-Kunststoff-Schlauch, verwendet werden, wobei in den Schlauch zeitlich getrennt Sauerstoff und Impfmaterial und/oder Sauerstoff und Rest- und/oder Abfallstoff eingeleitet werden. Vorzugsweise kann dabei während des Animpfschritts und/oder während des Inkubationsschritts ein Schlauchinnendruck variiert werden, insbesondere indem für einen Zeitraum ein Durchfluss von Impfmaterial und/oder von Rest- und/oder Abfallstoff durch den Schlauch erhöht wird und/oder indem für einen Zeitraum ein Volumenstrom von Sauerstoff durch den Schlauch erhöht wird. Die poröse Schlauchform des Trägerelements hat den Vorteil, dass eine relativ große Ansiedlungsfläche für die Mikroorganismen geschaffen wird. Insbesondere durch eine offenporige Ausgestaltung kann eine durch den Schlauch gepresste Suspension zusätzlich auch über die Schlauchwandung durchdringende Poren austreten. Somit ist einerseits eine besonders gute Animpfung sämtlicher Ansiedlungsflächen und zudem auch eine besonders gute Sauerstoff- und Nährstoffversorgung der Bakterien des Biofilms möglich. Insbesondere kann der Schlauch flexibel, also dehnbar, ausgestaltet sein, was den Vorteil hat, dass bei einer Druckerhöhung innerhalb des Schlauchs die Porendurchmesser ausgeweitet werden können. Somit kann zumindest für kurze Zeit eine erhöhte Durchflussrate durch die Poren erreicht werden, beispielsweise um eine bessere Umwälzung der Suspension auch innerhalb des Schlauchs zu erreichen.

Um eine besonders gute Benetzung des Trägermaterials erreichen zu können, kann das Trägerelement während des Animpfschritts vollständig in das Impfmaterial eingetaucht sein und/oder das Trägerelement während des Inkubationsschritt vollständig in den Rest- und/oder Abfallstoff eingetaucht sein.

Die Erfindung betrifft zudem eine organische Nährstofflösung, insbesondere einen organischen Pflanzendünger, hergestellt durch das Verfahren, wie es hierin beschrieben und beansprucht ist, und/oder in einem Bioreaktor, wie er hierin beschrieben und beansprucht ist, mit einem Anteil von mindestens 10%, insbesondere mindestens 25%, vorzugsweise mindestens 50%, weiter bevorzugt mindestens 75%, pflanzenverfügbarem mineralisiertem Stickstoff bezogen auf den Gesamtstickstoffgehalt der organischen Nährstofflösung. Vorzugsweise wobei ein Nitratanteil am pflanzenverfügbaren, mineralisierten Stickstoff höher als ein Ammoniumanteil ist. Weiter bevorzugt dass in der organischen Nährlösung ein NO3:NH4⁺-Verhältnis von mindestens 2:1, insbesondere von mindestens 3:1, insbesondere von mindestens 10:1, vorzugsweise von mindestens 25:1, weiter bevorzugt von mindestens 50:1, vorliegt. Die organische Nährstofflösung hat den Vorteil, dass sie im Gegensatz zu industriell hergestellten Mineraldüngern auch im biologischen Landbau einsetzbar ist. Rein aus organischen Rest- und/oder Abfallstoffen hergestellte organische Pflanzendünger mit einer derart hohen Stickstoff-Mineralisationsrate sind bisher nicht bekannt.

Vorzugsweise handelt es sich bei der organischen Nährstofflösung um einen Flüssigdünger.

Damit die organische Nährstofflösung im biologischen Landbau eingesetzt werden kann, ist es erforderlich, dass der pflanzenverfügbare mineralisierte Stickstoff ausschließlich oder im Wesentlichen aus organisch gebundenem Stickstoff umgesetzt ist und/oder dass die organische Nährstofflösung frei von industriell hergestelltem mineralischem Dünger ist. Als industriell hergestellter mineralischer Dünger zählen zum Beispiel mineralische Salze, die durch chemische oder physikalische Aufbereitung aus vor allem im Bergbau abgebauten Rohstoffen gewonnen werden, insbesondere also Stickstoffdünger, die keinen organischen Ursprung haben. Als organischer Rest-und/oder Abfallstoff, der als Ausgangstoff zur Herstellung der organischen Nährstofflösung verwendet wird, können beispielsweise pflanzliche und/oder tierische Abfälle, Gärreste, insbesondere aus Biogasanlagen, Gülle, Jauche, Stallmist, organische sekundäre Rohstoffe aus der Lebensmittel-, Genuss- und Futtermittelindustrie verwendet werden.

Die Erfindung betrifft weiter ein Substratmaterial zur Kultivierung von Pflanzen mit einem Biofilm mit ammonifizierenden und/oder nitrifizierenden Bakterien, hergestellt durch das Verfahren, wie es hierin beschrieben und beansprucht ist, und/oder mittels des Bioreaktors, wie er hierin beschrieben und beansprucht ist. Vorzugsweise kann das Trägerelement Poren und/oder Hohlräume mit einem Durchmesser von 10 µm bis 100 µm aufweisen.

Gemäß einer vorteilhaften Weiterbildung des Substratmaterials kann das Trägerelement als ein poröser Schlauch ausgestaltet sein. Vorzugsweise kann das Trägerelement als ein Schlauch hergestellt aus einer Kunststoff-Gummi-Mischung ausgestaltet sein. Hierbei wird Bezug auf die Ausführungsmöglichkeiten des Trägerelements des Bioreaktors genommen, die auch in Bezug auf das Substratmaterial Anwendung finden können.

Gemäß einer weiteren vorteilhaften Weiterbildung des Substratmaterials kann das Trägerelement aus einem Mineral, insbesondere aus Zeolith, hergestellt sein. Insbesondere kann das Trägerelement als Zeolith-Granulat ausgestaltet sein. Zeolith ist besonders gut als Bodenhilfsstoff geeignet, da es aufgrund seiner porösen Ausgestaltung eine besonders große Oberfläche aus inneren und äußeren Oberflächen aufweist, die als Ansiedlungsfläche dienen können. Somit weist das Zeolith trotz der relativ großen Ansiedlungsfläche dennoch einen relativ geringen Platzbedarf auf.

Um Pflanzen, die in Kontakt oder in der Nähe des Substratmaterials kultiviert werden, besser vor Nährstoff-und/oder Wassermangel schützen zu können, kann das Trägerelement eine Schwammwirkung aufweisen, durch welche das Substratmaterial Flüssigkeiten speichern kann. Dies kann beispielsweise dadurch erreicht werden, dass das Trägerelement zumindest teilweise aus einem aufgeschäumten Material, insbesondere aus einem aufgeschäumten Kunststoff hergestellt ist.

Die Erfindung betrifft außerdem die Verwendung ammonifizierender und/oder nitrifizierender Bakterien in Form eines Biofilms auf einem Trägerelement, insbesondere in einem Bioreaktor, wie er hierin beschrieben und beansprucht ist, zur Umwandlung von organischen Rest- und/oder Abfallstoffen in eine organische Nährstofflösung mit einem Anteil von mindestens 10%, insbesondere mindestens 25%, vorzugsweise mindestens 50%, weiter bevorzugt mindestens 75%, pflanzenverfügbarem mineralisiertem Stickstoff bezogen auf den Gesamtstickstoffgehalt der Nährstofflösung, insbesondere mittels einer Durchführung des Verfahrens, wie es hierin beschrieben und beansprucht ist. Vorzugsweise liegt dabei in der fertigen organischen Nährlösung ein N03:NH4+-Verhältnis von mindesten 2:1, insbesondere von mindestens 3:1, insbesondere von mindestens 10:1, vorzugsweise von mindestens 25:1, weiter bevorzugt von mindestens 50:1, vor.

Die Erfindung betrifft zudem die Verwendung der organischen Nährstofflösung, wie sie hierin beschrieben und beansprucht ist, zur Düngung von Pflanzen, insbesondere zur Düngung von Pflanzen die nach Kriterien des ökologischen Landbaus angebaut werden. Durch die organische Nährstofflösung kann eine gerade im letzten Wachstumsdrittel von Nutzpflanzen auftretende Bodenauslaugung besser ausgeglichen werden als mit herkömmlichen organischen Pflanzendüngern.

Gemäß einer vorteilhaften Ausgestaltung kann die organische Nährstofflösung in hydroponischen Anbausystemen, insbesondere in erdlosen und/oder in erdarmen hydroponischen Anbausystemen, verwendet werden. Im Gegensatz zu anderen rein organischen Pflanzendüngern eignet sich die organische Nährstofflösung auch für den Einsatz in erdlosen und/oder in erdarmen Anbausystemen, da der Anteil an pflanzenverfügbarem Stickstoff, insbesondere an pflanzenverfügbarem Nitrat, deutlich höher als bei herkömmlichen organischen Pflanzendüngern liegt.

Die Erfindung betrifft weiter die Verwendung des Substratmaterials, wie es hierin beschrieben und beansprucht ist, zur Kultivierung von Pflanzen. Insbesondere zur Kultivierung von Nutzpflanzen. Vorzugsweise kann das Substratmaterial unter eine Pflanzenerde, insbesondere als Bodenhilfsstoff, gemischt werden. Insbesondere kann es unter einen Ackerboden eines Feldes gemischt werden. Somit ist es möglich, die natürlicherweise im Boden ablaufenden Umwandlungsprozesse der Ammonifikation und/oder der Nitrifikation zu beschleunigen. Dadurch kann im Boden organisch gebundener Stickstoff schneller zu pflanzenverfügbarem Stickstoff umgewandelt werden. Somit lassen sich die Fruchtbarkeit von natürlichen Böden und die Anbauerträge steigern.

Alternativ oder ergänzend kann es vorgesehen sein, dass das Substratmaterial als ein Verankerungsmaterial, insbesondere in einem hydroponischen Anbausystem, verwendet wird. Das Substratmaterial kann daher dazu dienen, dass sich Pflanzen daran festhalten können, wenn die an sich dafür notwendige Erde nicht oder in nicht ausreichender Form vorhanden ist.

Gemäß einer bevorzugten Weiterbildung können die Pflanzen über ihre Wurzeln zumindest teilweise in direktem Kontakt mit einer Außenseite des Trägerelements des Substratmaterials stehen. Insbesondere kann durch das Trägerelement eine organische Nährstofflösung, insbesondere wie hierin beschrieben und beansprucht, geleitet werden. Vorzugsweise kann die organische Nährlösung dann durch Poren in einer Trägerelementwandung von einer Innenseite des Trägerelements zu der Außenseite des Trägerelements diffundieren und/oder gepresst werden. Dies ermöglicht es, dass eine besonders gute Nährstoffversorgung der Pflanzen möglich ist. So ist es beispielsweise möglich, das Trägerelement in einem Ackerboden zu verlegen. Weiter kann das Trägerelement in dieser Form auch, wie bereits zuvor ausgeführt wurde, in einem erdarmen oder erdlosen System verwendet werden.

Gemäß einer besonders bevorzugten Weiterbildung können die Pflanzen über ihre Wurzeln zumindest teilweise in direktem Kontakt mit einer Außenseite des Trägerelements des Substratmaterials stehen, wobei durch das Trägerelement ein organischer Rest- und/oder Abfallstoff geleitet wird, wobei durch die Bakterien des Biofilms organisch gebundener Stickstoff des Rest- und/oder Abfallstoffes in mineralisierten Stickstoff umwandelt wird, wobei der mineralisierte Stickstoff durch Poren in einer Trägerelementwandung von einer Innenseite des Trägerelements zu der Außenseite des Trägerelements diffundiert und/oder gepresst wird, und wobei die mit der Außenseite des Trägerelements zumindest teilweise in Kontakt stehenden Wurzeln der Pflanzen den pflanzenverfügbaren, mineralisierten Stickstoff aufnehmen. Somit kann der organische Rest- und/oder Abfallstoff direkt zur Pflanzenversorgung eingesetzt werden, ohne dass vorher eine separat durchgeführte Umwandlung in organische Nährstofflösung notwendig ist. Dabei hat sich überraschender Weise gezeigt, dass die Pflanzen aufgrund der effizienten Umsetzung des organisch gebundenen Stickstoffs in pflanzenverfügbaren Stickstoff durch den Biofilm auf dem Trägerelement ausreichend mit Stickstoff versorgt werden.

Die Erfindung betrifft weiter ein Set aus einem Bioreaktor, wie er hierin beschrieben und beansprucht ist, und einem Impfmaterial zum Animpfen des Trägerelements und zur Ausbildung eines Biofilms mit ammonifizierenden und/oder nitrifizierenden Bakterien.

Die Erfindung betrifft zudem ein Set aus einem Trägerelement und einem Impfmaterial zum Animpfen des Trägerelements und zur Ausbildung eines Biofilms mit ammonifizierenden und/oder nitrifizierenden Bakterien, insbesondere zur Ausführung eines Verfahrens, wie es hierin beschrieben und beansprucht ist, und/oder in einem Bioreaktor, wie er hierin beschrieben und beansprucht ist, und/oder zur Verwendung wie sie hierin beschrieben und beansprucht ist.

Die Erfindung wird nun anhand von Ausführungsbeispielen näher erläutert, ist aber nicht auf diese Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen der Ausführungsbeispiele.

### Ausführungsbeispiele

### 1. Beispiel

Zur Durchführung des Verfahrens zur Herstellung einer organischen Nährstofflösung wird bei den nachfolgenden Beispielen ein Bioreaktor genutzt, wie er hierin beschrieben und beansprucht ist.

In den Aufnahmeraum des Reaktionsbehälters werden zunächst Gärreste, gewonnen aus der Fermentierung von Bioabfällen zusammengesetzt aus getrennter Sammlung aus privaten Haushalten (92 %), pflanzlichen Stoffen aus der Lebens-, Genuss- und Futtermittelherstellung, pflanzlichen Stoffen aus Garten- und Landschaftsbau, aus der Forstwirtschaft, Fett und Fettrückstände, mit einem geringen Anteil pflanzenverfügbaren Stickstoffes (weniger als 1 %) beigefügt.

Alternativ oder ergänzend können vergorene Zuckerrübenmelasse (Vinasse) als Reststoff der Lebensmittel- und Futtermittelindustrie mit einem Anteil verfügbaren Stickstoffes von weniger als 0,5 % beigefügt werden.

Die Beimpfung der Trägerelemente erfolgt wie zuvor erläutert wurde jeweils in einem ersten Behälter.

Einem zweiten Behältnis wird dann das beimpfte Kunststoffträgermaterial in Spanform mit einem Gewicht von 200 g und einer geschätzten Kunststoffkontaktfläche von 1,76 m² hinzugefügt und mit 720 ml des organischen flüssigen Gärrestes und 11 l Wasser aufgefüllt.

Der Gärrest besitzt folgende Zusammensetzung:
0,46 % N Gesamtstickstoff
0,18 % N Ammoniumstickstoff
0,12 % P₂O₅ Gesamtphosphat
0,42 % K20 Gesamtkaliumoxid
0,0029 % Zn Gesamtzink
Nebenbestandteile:
0,11 % MgO Gesamtmagnesiumoxid
0,04 % S Schwefel
0,66 % CaO Basisch wirksame Bestandteile
6,41 % Organische Substanz
Rohdichte 1040 kg/m³
pH-Wert 8,4

Damit beträgt der Gesamtstickstoff der Ausgangslösung rechnerisch 294 mg/l. Das eingebrachte Gärrestematerial zusammen mit 11 l Wasser weist die Ausgangswerte (gemessen mit MQuant™ Teststreifen der Fa. Merck KgaA, 64271 Darmstadt, Germany) auf:
pH = 7,4
NH₄ = 200 mg/l entspricht NH4-N = 155 mg/l
NO₃ = 0 mg/l

Während des Inkubationsschritt wird die Flüssigkeit auf 25 °C temperiert und für 6 Minuten pro Stunde und 14 Stunden am Tag Luft eingeblasen. Das entspricht einer Gesamtluftmenge von 25,2 m³ pro Tag.

Nach 5 Tagen beträgt der NO₃-Wert 250 mg/l (entspricht 56 mg/l NO₃-N) und der NH4-Wert ist auf 90 mg/l (entspricht 70 mg/l NH₄-N) gesunken. Nach 9 Tagen erreicht der NO₃-Wert sein Maximum von 1000 mg/l (226 mg/l NO₃-N) und der NH₄-Wert liegt bei 15 mg/l (12 mg/l NH₄-N). Zu diesem Zeitpunkt liegen 81 % (238 mg/l) des in der Ausgangslösung enthaltenen Gesamtstickstoffes in pflanzenverfügbarer Form vor. Dabei liegt der Stickstoff in einem NO₃ zu NH₄-Verhältnis von 50:1 vor. In der Ausgangslösung liegen 39 % des Gesamtstickstoffes zu 100 % als NH₄ direkt pflanzenverfügbar vor.

In Mitteleuropa beträgt die mittlere jährliche Mineralisationsrate im Boden, abhängig von Temperatur und Bodenfeuchte etwa 1-2 % des organischen Stickstoffes. Bei einer Mineralisationsrate von 2 % würde der berechnete GesamtStickstoff von 294 mg/l im Boden mit 5,88 mg/l pro Jahr mineralisieren. Nach 9 Tagen wären 0,145 mg/l mineralisiert. Daraus ergäbe sich bei dieser Nutzung eine mehr als 1500fach höhere Mineralisationsrate verglichen zur Bodenmineralisation.

### 2. Beispiel

In diesem Fall wird Vinasse als organischer Rest- und/oder Abfallstoff eingesetzt.

Dem zweiten Behältnis wird das beimpfte Kunststoffträgermaterial in Spanform mit einem Gewicht von 150 g und einer geschätzten Kunststoffkontaktfläche von 1,32 m² hinzugefügt und der Behälter wird mit 60 ml Vinasse und 93 l Wasser aufgefüllt.

Die Vinasse besitzt folgende Zusammensetzung:
4,5 % N Gesamtstickstoff
0,5 % N verfügbarer Stickstoff
6 % K₂O Gesamtkaliumoxid
Nebenbestandteile:
1,5 % S wasserlöslicher Schwefel
2,5 % Na wasserlösliches Natrium
48 % Organische Substanz
Dichte 1360 kg/m³

Damit beträgt der Gesamtstickstoff rechnerisch 40 mg/l. Die eingebrachte Vinasse zusammen mit 93 l Wasser weist die Ausgangswerte (gemessen mit Mquant Teststreifen der Fa. Merck KgaA, 64271 Darmstadt, Germany) auf:
pH = 6,8
NH4 = 20 mg/l entspricht NH4-N = 16 mg/l
N03 = 5 mg/l entspricht N03-N = 1,2 mg/l

Während des Inkubationsschritts wird die Flüssigkeit auf 25 °C temperiert und für 10 Minuten pro Stunde und 14 Stunden am Tag Luft eingeblasen. Das entspricht einer Gesamtluftmenge von 42 m³ pro Tag.

Nach 5 Tagen beträgt der NH₄-Wert 45 mg/l (entspricht 35 mg/l NH₄-N) und nach 9 Tagen einen Maximalwert von 80 mg/l (entspricht 62 mg/l NH₄-N). Nach 9 Tagen liegt der NO₃-Wert bei 3 mg/l und steigt auf einen Maximalwert von 240 mg/l (50 mg/l NO₃-N) am 11. Tag. Zu diesem Zeitpunkt liegt der NH₄-Wert bei 8 mg/l (6 mg/l NH₄-N).

Zu diesem Zeitpunkt liegen 140 %* (56 mg/l) des in der Ausgangslösung enthaltenen Gesamtstickstoffes in pflanzenverfügbarer Form vor. Dabei liegt der Stickstoff in einem NO₃ zu NH₄-Verhältnis von 8:1 vor. In der Ausgangslösung liegen 11 % des Gesamtstickstoffes in einem NO₃ zu NH₄-Verhältnis von 1:10 direkt pflanzenverfügbar vor.

In Mitteleuropa beträgt die mittlere jährliche Mineralisationsrate im Boden, abhängig von Temperatur und Bodenfeuchte etwa 1-2 % des organischen Stickstoffes. Bei einer Mineralisationsrate von 2 % würde der berechnete GesamtStickstoff von 40 mg/l im Boden mit 0,8 mg/l pro Jahr mineralisieren. Nach 9 Tagen wären 0,02 mg/l mineralisiert. Daraus ergäbe sich bei dieser Nutzung eine mehr als 2500fach höhere Mineralisationsrate verglichen zur Bodenmineralisation.

*Der Gesamtstickstoff wird nach der Methode von Kjeldahl ermittelt. Bestimmt wird hierbei maßgeblich der im Proteinanteil enthaltene Stickstoff, wobei die Schwankungen des Stickstoffanteils in Abhängigkeit der Aminosäurezusammensetzung nicht erfasst wird. Daraus folgt, dass der reale Stickstoffanteil im Ausgangsmaterial höher ist als der nach Kjeldahl analytisch ermittelte Wert.

### 3. Beispiel

Als Trägermaterial wird hier ein poröser Schlauch eingesetzt.

Durch das beimpfte Trägerelement in Schlauchform werden Gärreste, gewonnen aus der Fermentierung von Bioabfällen zusammengesetzt aus getrennter Sammlung aus privaten Haushalten (92 %), pflanzliche Stoffe aus der Lebens-, Genuss- und Futtermittelherstellung, pflanzliche Stoffe aus Garten- und Landschaftsbau, aus der Forstwirtschaft, Fett und Fettrückstände mit einem geringen Anteil verfügbaren Stickstoffes (weniger als 1 %), geleitet.

Alternativ oder ergänzend kann durch das das beimpfte Trägerelement in Schlauchform vergorene Zuckerrübenmelasse (Vinasse) als Reststoff der Lebensmittel- und Futtermittelindustrie mit einem Anteil verfügbaren Stickstoffes von weniger als 0,5 % geleitet werden.

Auf den Poren des Trägerelements in Schlauchform tritt jeweils eine Nährstofflösung mit sofort pflanzenverfügbarem, mineralsierten N aus, die von Pflanzwurzeln, die mit dem Substrat in Kontakt sind als sofort pflanzenverfügbare, mineralische Nährstofflösung aufgenommen wird.

Dazu werden 720 ml werden organischer flüssiger Gärrest und 13 l Wasser aufgefüllt.

Der Gärrest besitzt folgende Zusammensetzung:
0,46 % N Gesamtstickstoff
0,18 % N Ammoniumstickstoff
0,12 % P₂O₅ Gesamtphosphat
0,42 % K20 Gesamtkaliumoxid
0,0029 % Zn Gesamtzink
Nebenbestandteile:
0,11 % MgO Gesamtmagnesiumoxid
0,04 % S Schwefel
0,66 % CaO Basisch wirksame Bestandteile
6,41 % Organische Substanz
Rohdichte 1040 kg/m3
pH-Wert 8,4

Damit beträgt der Gesamtstickstoff der Ausgangslösung rechnerisch 251 mg/l. Das eingebrachte Gärrestematerial weist die Ausgangswerte (gemessen mit Mquant Teststreifen der Fa. Merck KGaA, 64271 Darmstadt, Germany) auf:
pH = 7,2
NH4 = 180 mg/l entspricht 140 mg/l NH₄-N
N03 = 0 mg/l

Bei Messung der abgetropften aufgefangenen Flüssigkeit (A) und an der äußeren Oberfläche des beimpften Trägerelements in Schlauchform (B) ergeben sich folgende Werte nach 3 Tagen:

| | N03 (N03-N) mg/l | NH4 (NH4-N) mg/l | N03 zu NH4 Verhältnis |
|---|---|---|---|
| A | 40 (9) | 190 (148) | 1:4,75 |
| B | 300 (69) | 50 (34) | 6:1 |

und nach 5 Tagen:

| | N03 (N03-N) mg/l | NH4 (NH4-N) mg/l | N03 zu NH4 Verhältnis |
|---|---|---|---|
| A | 50 (11) | 180 (140) | 1:3,6 |
| B | 500 (113) | 80 (62) | 6,25:1 |

Bei einem unbeimpften Trägerelement in Schlauchform, dass parallel mit der Ausgangslösung versorgt wurde, ergeben sich sowohl nach 3 als auch nach 5 Tagen die Ausgangswerte bei A und B Messung.

### 4. Beispiel

Es werden durch das beimpfte Trägerelement in Schlauchform 25 ml der organischen Vinasse und 10 l Wasser aufgefüllt.

Die Vinasse besitzt folgende Zusammensetzung:
4,5 % N Gesamtstickstoff
0,5 % N verfügbarer Stickstoff
6 % K₂O Gesamtkaliumoxid
Nebenbestandteile:
1,5 % S wasserlöslicher Schwefel
2,5 % Na wasserlösliches Natrium
48 % Organische Substanz
Dichte 1360 kg/m³

Damit beträgt der Gesamtstickstoff der Ausgangslösung rechnerisch 153 mg/l. Die eingebrachte Vinasse zusammen mit 10 l Wasser weist die Ausgangswerte (gemessen mit Mquant Teststreifen der Fa. Merck KgaA, 64271 Darmstadt, Germany) auf:
pH = 6,5
NH4 = 15 mg/l entspricht 12 mg/l NH₄-N
N03 = 0 mg/l

Bei Messung der abgetropften aufgefangenen Flüssigkeit (A) und an der äußeren Oberfläche des beimpften Pflanzsubstrates in Schlauchform (B) ergeben sich folgende Werte nach 1 Tag:

| | N03 (N03-N) mg/l | NH4 (NH4-N) mg/l | N03 zu NH4 Verhältnis |
|---|---|---|---|
| A | 35 (27) | 5 (4) | 7:1 |
| B | 35 (27) | 5 (4) | 7:1 |

nach 7 Tagen:

| | N03 (N03-N) mg/l | NH4 (NH4-N) mg/l | N03 zu NH4 Verhältnis |
|---|---|---|---|
| A | 5 (1) | 80 (62) | 1:16 |
| B | 90 (21) | 8 (6) | 11:1 |

nach 14 Tagen:

| | NO₃ (NO₃-N) mg/l | NH₄ (NH₄-N) mg/l | NO₃ zu NH₄ Verhältnis |
|---|---|---|---|
| A | 20 (5) | 150 (117) | 1:7,5 |
| B | 400 (92) | 70 (55) | 5,7:1 |

Bei einem unbeimpften Trägerelement in Schlauchform, dass parallel mit der Ausgangslösung versorgt wurde, ergeben sich nach 1, 7 und nach 14 Tagen die Ausgangswerte bei A und B Messung.

### 5. Beispiel

Als Trägerelement wird Zeolith-Granulat verwendet.

Das beimpfte Zeolith-Granulat wird einem Pflanzboden beigemischt. Gärreste, gewonnen aus der Fermentierung von Bioabfällen zusammengesetzt aus getrennter Sammlung aus privaten Haushalten (92 %), pflanzlichen Stoffen aus der Lebens-, Genuss- und Futtermittelherstellung, pflanzlichen Stoffen aus Garten- und Landschaftsbau, aus der Forstwirtschaft, Fett und Fettrückstände mit einem geringen Anteil verfügbaren Stickstoffes (weniger als 1 %) werden dem Zeolith-Boden-Gemisch beigefügt.

Alternativ oder ergänzend können vergorene Zuckerrübenmelasse (Vinasse) als Reststoff der Lebensmittel- und Futtermittelindustrie mit einem Anteil verfügbaren Stickstoffes von weniger als 0,5 % dem Zeolith-Boden-Gemisch beigefügt werden.

Dem zweiten Behältnis wird das beimpftes Zeolitht-Granulat mit einem Gewicht von 400 g und einer geschätzten Kontaktfläche von 21,6 m² hinzugefügt und mit 440 ml des organischen flüssigen Gärrestes und 28 l Wasser aufgefüllt.

Der Gärrest besitzt folgende Zusammensetzung:
0,46 % N Gesamtstickstoff
0,18 % N Ammoniumstickstoff
0,12 % P₂O₅ Gesamtphosphat
0,42 % K₂O Gesamtkaliumoxid
0,0029 % Zn Gesamtzink
Nebenbestandteile:
0,11 % MgO Gesamtmagnesiumoxid
0,04 % S Schwefel
0,66 % CaO Basisch wirksame Bestandteile
6,41 % Organische Substanz
Rohdichte 1040 kg/m³
pH-Wert 8,4

Damit beträgt der Gesamtstickstoff der Ausgangslösung rechnerisch 74 mg/l. Das eingebrachte Gärrestematerial zusammen mit 28 l Wasser weist die Ausgangswerte (gemessen mit Mquant Teststreifen der Fa. Merck KgaA, 64271 Darmstadt, Germany) auf:
pH = 7,4
NH₄ = 80 mg/l entspricht 56 mg/l NH₄-N
NO₃ = 5 mg/l entspricht 1 mg/l NO₃-N

Nach 5 Tagen beträgt der NO₃-Wert 75 mg/l (entspricht 17 mg/l NO₃-N) und der NH₄-Wert ist auf 5 mg/l (entspricht 4 mg/l NH₄-N) gesunken. Zu diesem Zeitpunkt liegen 28 % (21 mg/l) des in der Ausgangslösung enthaltenen Gesamtstickstoffes in pflanzenverfügbarer Form vor. Dabei liegt der Stickstoff in einem NO₃ zu NH₄-Verhältnis von 15:1 vor. In der Ausgangslösung liegen 77 % des Gesamtstickstoffes in einem NO₃ zu NH₄-Verhältnis von 1:16 vor.

Nachfolgend wir der Bioreaktor anhand der Figuren genauer beschrieben.

Es zeigt:
- Fig. 1: eine erste Ausführungsvariante eines Bioreaktors mit mehreren, zu einem Knäul zusammengefügten Kunststoffspänen als Trägerelemente,
- Fig. 2: eine zweite Ausführungsvariante eines Bioreaktors mit mehreren, als Zeolith-Granulat ausgebildeten Trägerelementen, die in einem Textilbeutel innerhalb des Aufnahmeraums des Bioreaktors angeordnet sind,
- Fig. 3: eine dritte Ausführungsvariante eines Bioreaktors mit drei jeweils als poröse Schläuche ausgebildeten Trägerelementen, die parallel zueinander, in einer Leitung innerhalb des Aufnahmeraums angeschlossen sind.

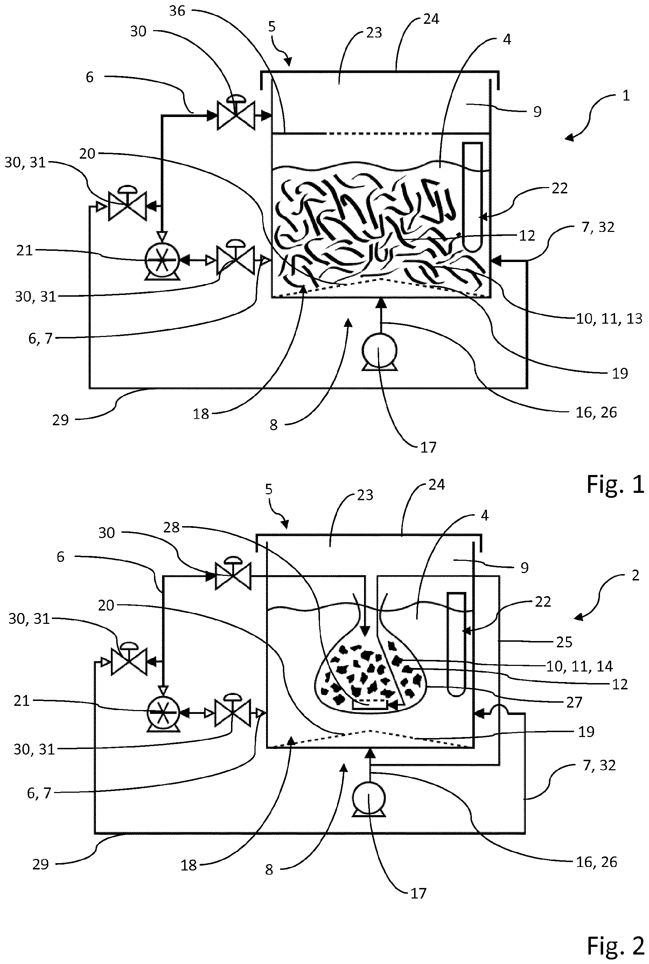

In den Figuren 1 bis 3 sind drei unterschiedliche Ausführungsbeispiele eines Bioreaktors gezeigt, die im Ganzen jeweils als 1, 2 oder 3 bezeichnet sind. Die Bioreaktoren 1, 2 und 3 sind zur Umwandlung von organischen Rest- und/oder Abfallstoffen in eine organische Nährstofflösung mit einem relativ hohen Anteil von pflanzenverfügbarem mineralisiertem Stickstoff eingerichtet.

Mittels des Bioreaktors 1, 2, 3 kann somit ein Verfahren zur Herstellung der organischen Nährstofflösung durchgeführt werden, wobei dabei ein Anteil von mindestens 10% pflanzenverfügbarem Stickstoff bezogen auf den Gesamtstickstoffgehalt der Nährstofflösung vorgesehen ist. Des Weiteren soll bei dem pflanzenverfügbaren mineralisierten Stickstoff der Nitratanteil höher als der Ammoniumanteil sein.

Der Bioreaktor 1, 2, 3 weist einen Reaktionsbehälter 5 auf, in welchen eine Zuleitung 6 einmündet und eine Ableitung 7 ausmündet. Über die Zuleitung 6 kann eine Suspension 4 in den Reaktionsbehälter eingeleitet werden und über die Ableitung 7 kann die Suspension nach Durchlaufen des Reaktionsbehälters 5 wieder ausgeleitet werden.

Der Bioreaktor 1, 2, 3 weist eine Belüftungseinrichtung 8 auf, über welche vorzugsweise in Form von Luft, Sauerstoff in den Reaktionsbehälter 5 einleitbar ist. Bei den dargestellten Ausführungsbeispielen der Figuren 1 bis 3 weist die Belüftungseinrichtung 8 einen Kompressor 17 auf. Über eine Gaszuführleitung 16 kann mittels des Kompressors 17 Sauerstoff in Form von Luft in den Reaktionsbehälter 5 eingeleitet werden.

Die drei Bioreaktoren 1, 2 und 3 unterscheiden sich im Wesentlichen durch unterschiedliche Trägerelemente 10, die jeweils innerhalb eines Aufnahmeraums 9 des Reaktionsbehälters 5 angeordnet sind. Die Trägerelemente 10 sind derart innerhalb des Aufnahmeraums 9 angeordnet, dass eine Umspülung der Trägerelemente 10 mittels der über die Zuleitung 6 eingeleiteten Suspension 4 möglich ist. Die Suspension 4 kann beispielsweise ein organischer Rest- und/oder Abfallstoff, wie zuvor beschrieben, und/oder ein organisches Impfmaterial, wie zuvor beschrieben, sein. Ferner sind die Trägerelemente 10 auch derart angeordnet, dass der mittels der Belüftungseinrichtung 8 eingeleitete Sauerstoff die Trägerelement 10, vorzugsweise im Wesentlichen allseitig, umströmt.

Die Trägerelemente 10 weisen eine besonders große Oberfläche im Verhältnis zu ihrem Volumen auf. Die Oberflächen der Trägerelemente 10 sind dabei als Ansiedlungsflächen 11 für die Ausbildung eines Biofilms 12, der zumindest teilweise aus ammonifizierenden und/oder nitrifizierenden Bakterien besteht, ausgebildet. Die Ansiedlungsflächen 11 sind daher beispielsweise rauer ausgestaltet, als eine Innenseite einer Reaktionsbehälterwandung. Somit können sich die Mikroorganismen des Biofilms 12 besonders gut auf den Ansiedlungsflächen 11 anhaften und darauf wachsen. Dies ermöglicht es, dass die ammonifizierenden und/oder nitrifizierenden Bakterien im Wesentlichen an den Ansiedlungsflächen 11 einen Biofilm 12 ausbilden, da hier ideale Wachstumsbedingungen erzeugbar sind.

Die Trägerelemente 10 der verschiedenen Ausführungsbeispiele der Figuren 1 bis 3 sind zum Teil aus unterschiedlichen Materialien oder Kombinationen von mehreren Materialien hergestellt.

Die Trägerelemente 10 des Bioreaktors 1 aus Figur 1 sind als Späne 13 aus Kunststoff hergestellt. Beispielsweise können diese Späne 13 als Abfallprodukte bei einer zerspanenden Bearbeitung eines Kunststoffrohlings entstehen. Besonders vorteilhaft kann es dabei sein, wenn die Späne aus einem Thermoplast, wie beispielsweise aus Polypropylen und/oder Polyethylen bestehen. Die Vielzahl von Spänen 13 ist dabei in ungeordneter Weise innerhalb des Aufnahmeraums 9 des Reaktionsbehälters 5 angeordnet. Dadurch entsteht ein regelrechtes Verknäulen der Späne 13. Da die Späne 13 eine gekrümmte Ansiedlungsfläche 11 aufweisen, die beispielsweise aufgrund einer mäandernden und/oder spiralförmigen und/oder welligen Form der Späne 13 entstehen kann, kann in einfacher Weise verhindert werden, dass die Ansiedlungsflächen 11 einzelner Späne 13 aneinander anhaften oder anliegen. Ein Anhaften oder Anliegen ist daher nachteilig, weil ein Gasaustausch und/oder eine Umspülung nicht mehr gewährleistbar wäre/wären und sich daher ein Biofilm aus anaeroben Bakterien ausbilden könnte. Dies könnte genau zum ungewollten Effekt führen, dass insbesondere Denitrifizierungsprozesse stattfinden.

Bei dem Bioreaktor 2 aus Figur 2 sind die Trägerelemente 10 als Zeolithgranulate 14 ausgebildet. Um ein Ablagern der Granulate 14 in schlecht durchströmten oder schlecht belüfteten Bereichen des Aufnahmeraums 9 verhindern zu können, sind die als Granulate 14 ausgebildeten Trägerelemente 10 in einer als Textilbeutel ausgestalteten Sammeleinheit 27 angeordnet. Die Sammeleinheit 27 kann innerhalb des Aufnahmeraums mittels einer Aufhängung fixierbar sein. Innerhalb der Sammeleinheit 27 ist eine weitere Belüftungseinrichtung 28 angeordnet, die über eine Bypassgasleitung 25 mit Sauerstoff versorgt werden kann. Des Weiteren führt eine innerhalb des Reaktionsbehälters 5 verlaufende Zuleitung 6 bis in die Sammeleinheit 27 und endet dort, wodurch die Suspension 4 direkt in die Sammeleinheit 27 einleitbar ist. Die Sammeleinheit 27 ist offenporig ausgestaltet, sodass die Suspension aus der Sammeleinheit 27 in den Aufnahmeraum 9 austreten kann. Die Bypassgasleitung 25 zweigt von einer Hauptgasleitung 26 ab, die mit der Belüftungseinrichtung 8 verbunden ist. Somit ist es möglich, Sauerstoff an zwei unterschiedlichen Orten in den Aufnahmeraum 9 einzuleiten, ohne dass ein zweiter Kompressor 17 erforderlich ist.

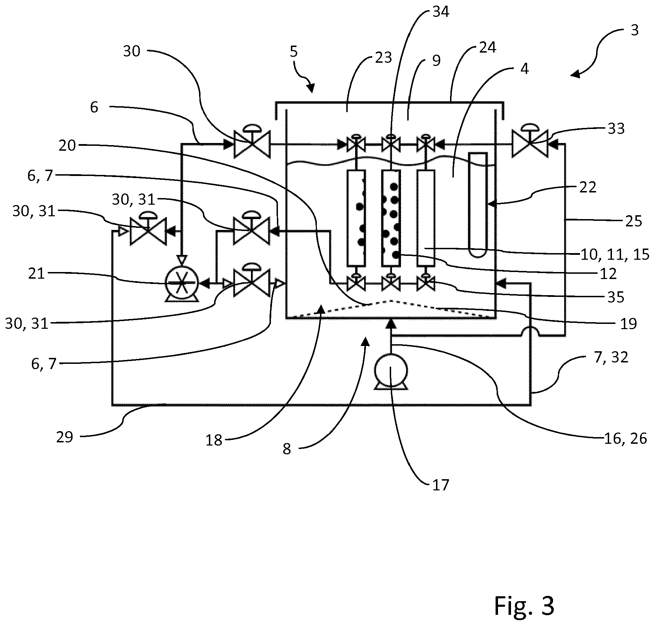

Der Bioreaktor 3 aus Figur 3 weist drei jeweils als poröse Schläuche 15 ausgebildete Trägerelemente 10 auf, die parallel zueinander in ein Leitungssystem innerhalb des Aufnahmeraums 9 integriert sind. Die Schläuche 15 sind jeweils mit der Zuleitung 6 und einer als Bypassgasleitung 25 ausgebildeten Gaszufuhrleitung 16 verbunden. Somit kann insbesondere zeitlich getrennt Suspension 4 und Sauerstoff in die Schläuche 15 eingeleitet werden. Um das Einleiten von Sauerstoff oder Suspension 4 in einen Schlauch 15 oder mehrere der Schläuche 15 zu unterbinden, ist in Strömungsrichtung in die Zuleitung 6 vor jedem Schlauch 15 ein Absperrventil 34 angeordnet.

Um einen Schlauchinnendruck in einem Schlauch 15 oder in mehreren Schläuchen 15 insbesondere unabhängig von den anderen Schläuchen 15 erhöhen zu können, ist in Strömungsrichtung der Suspension 4 nach jedem Schlauch 15 jeweils ein weiteres Absperrventil 35 angeordnet. Durch Absperrung eines Ventils 35 kann ein Austritt der Suspension aus dem Schlauch 15 über die innerhalb des Aufnahmeraums 9 verlaufende Ableitung 7 verhindert werden. Somit kann die Suspension 4 über die Poren in einer Schlauchwandung in den Aufnahmeraum 9 austreten. Da der Schlauch 15 vorzugsweise dehnbar ausgebildet ist, kann durch eine Druckerhöhung innerhalb des Schlauchs 15 eine Vergrößerung der Poren in der Schlauchwandung erreicht werden. Der Bioreaktor 3 weist eine weitere Ableitung 7 auf, über welche im Falle einer Absperrung der ersten Ableitung 7 Suspension 4 aus dem Aufnahmeraum 9 ausleitbar ist.

Die Belüftungseinrichtung 8 weist eine Belüftungsplatte 19 auf, die an einem Boden 18 des Reaktionsbehälters 5 des Bioreaktors 1, 2, 3 angeordnet ist. Die Belüftungsplatte 19 ist über eine Gaszufuhrleitung 16, insbesondere die Hauptgasleitung 26, mit dem Kompressor 17 verbunden. Die Belüftungsplatte 19 weist mehrere, gleichmäßig verteilte Belüftungsöffnungen 20 auf, über welche Sauerstoff in die Suspension 4 einströmen kann.

Der Bioreaktor 1, 2, 3 weist jeweils eine Pumpvorrichtung 21 auf, die insbesondere als Kreiselpumpe oder Umwälzpumpe ausgestaltet sein kann. Mittels der Pumpvorrichtung 21 ist es möglich, die Suspension 4 durch die Zuleitung 6 in den Reaktionsbehälter 5 zu pumpen und über die Ableitung 7 aus dem Reaktionsbehälter 5 abzusaugen.

Der Bioreaktor 1, 2, 3 weist somit einen aus den Zuleitungen 6, den Ableitungen 7 und dem Reaktionsbehälter 5 bestehenden Suspensionskreislauf 29 auf, in welchem die Suspension 4 mittels der Pumpvorrichtung 21 zirkulierbar ist. Die Pumpvorrichtung 21 ist derart eingerichtet, dass eine Suspensionsströmungsrichtung innerhalb des Reaktionsbehälters 5 und/oder innerhalb der Leitungen des Bioreaktors 1, 2, 3 umkehrbar ist. In Kombination mit mehreren Absperrventilen 30, 31, 34, 35 kann die Strömungsrichtung innerhalb des Reaktionsbehälters 5 eingestellt und variiert werden.

Von der Zuleitung 6 kann beispielsweise, wie in den Figuren 1 bis 3 gezeigt ist, eine Bypasszuleitung 32 abzweigen, die in den Aufnahmeraum 9 des Reaktionsbehälters 5 mündet. Wird die Suspensionsströmungsrichtung umgekehrt, kann eine Zuleitung 6 der mehreren Zuleitungen 6 zu einer Ableitung 7 umfunktioniert werden und/oder die Bypasszuleitung 32 zu einer Ableitung 7 umfunktioniert werden. Die Funktion der jeweiligen Leitung ist somit von der anhand der Pumpvorrichtung 21 vorgegebenen Strömungsrichtung der Suspension 4 abhängig. Allgemein kann jedoch gesagt werden, dass Zuleitungen 6 oberhalb oder zumindest auf gleicher Höhe der Ableitungen 7 in den Aufnahmeraum 9 des Reaktionsbehälters 5 münden. Somit kann eine bessere Umwälzung der Suspension innerhalb des Reaktionsbehälters 5 erreicht werden.

Um das Trägerelement 10 einfach aus dem Aufnahmeraum 9 herausnehmen zu können, weist der Bioreaktor 1, 2, 3 eine Öffnung 23 an einer Oberseite des Reaktionsbehälters 5 auf. Mittels einer als Deckel ausgebildeten Verschlusseinheit 24 kann diese Öffnung 23 flüssigkeitsdicht und/oder druckbeständig verschlossen werden, während der Bioreaktor 1, 2, 3 in Gebrauch ist.

Der Bioreaktor 1 weist im oberen Drittel des Aufnahmeraums 9 eine Zerteilereinheit 36 auf, über welche die Suspension 4 in mehrere Einzelstrahlen aufteilbar ist. Dadurch kann einerseits eine Zerteilung von aneinander anhaftenden Feststoffen und andererseits eine zusätzliche Durchlüftung der Suspension erreicht werden. Die Zerteilereinheit 36 kann beispielsweise als eine Zerteilerplatte ausgestaltet sein. Ferner kann diese Zerteilereinheit 36 auch bei den anderen Ausführungsvarianten der Figuren 2 und 3 oder mit den Merkmalen aus den Ansprüchen kombiniert werden.

Der Bioreaktor 1, 2, 3 weist weiter eine Heizvorrichtung 22 auf, mittels welcher der Aufnahmeraum 9 und/oder die darin enthaltene Suspension 4 auf eine gewünschte Temperatur aufwärmbar ist.

Wie in Figur 3 zu erkennen ist, kann es vorgesehen sein, dass in der Bypassgasleitung 25 ein Absperrventil 33 angeordnet ist. Dadurch kann verhindert werden, dass Sauerstoff in den Aufnahmeraum 9 des Reaktionsbehälters 5 eingeleitet wird, gleichzeitig jedoch eine Belüftung über die Belüftungsplatte 19 erfolgen kann.

Zur Erzeugung eines Biofilms 12 aus zumindest teilweise ammonifizierenden und/oder nitrifizierenden Bakterien wird aus einem partikelförmigen organischen Impfmaterial eine Suspension mit Wasser hergestellt. Als Impfmaterial kann dabei beispielsweise ein Wurmexkrement oder eine Wurmerde dienen. Weitere mögliche Impfmaterialien wurden zuvor bereits ausführlich beschrieben. Grundsätzlich kann gesagt werden, dass als Impfmaterial prinzipiell sämtliche organischen Stoffe geeignet sind, die proteolytische Bodenbakterien enthalten.

Für eine optimale Biofilmbildung wird das Impfmaterial durch Umwälzung und Verwirbelung durch Lufteinblasung mit dem Trägermaterial 10 in Kontakt gebracht. Das organische Impfmaterial weist eine erhöhte Konzentration von Bodenbakterien, Schleim und anderen Eiweißstoffen sowie anorganischen Mineralien auf, die mit abgestorbenen Bakterienmaterial behaftet sind. Diese Inhaltsstoffe unterstützen einerseits die Anhaftung der enthaltenen Bakterien auf dem Trägerelement 10 und damit die Biofilmbildung. Darüber hinaus dienen sie den Bakterien auch als Nährstoffe. Damit entsteht ein Trägerelement 10 mit einer vielfältigen, qualitativ und quantitativ veränderbaren und modifizierbaren Bakterienkultur bestehend aus einer Vielzahl von Bodenbakterien einschließlich ammounifizierender und nitrifizierender Bakterien.

Nach Ausbildung des Biofilms 12 auf dem Trägerelement 10 kann das Trägermaterial 10 aus einem ersten Reaktionsbehälter 5 entnommen werden und in einen weiteren Reaktionsbehälter 5 überführt werden. Anschließend kann eine Umwandlung eines organischen Rest- und/oder Abfallstoffes in eine organische Nährlösung mittels des Biofilms vorgenommen werden. Es ist allerdings auch denkbar, dass der Impfschritt und der Inkubationsschritt im selben Reaktionsbehälter 5 durchgeführt werden. Dabei empfiehlt es sich, das Impfmaterial vor Zugabe des organischen Rest- und/oder Abfallstoffes aus dem Reaktionsbehälter 5 zu entfernen.

Die Erfindung betrifft also einen Bioreaktor 1, 2, 3 und dessen Verwendung zur Umwandlung von organischen Rest- und/oder Abfallstoffen in eine organische Nährstofflösung mit einem Anteil von mindestens 10% pflanzenverfügbarem mineralisiertem Stickstoff bezogen auf den Gesamtstickstoffgehalt der Nährstofflösung, mit einem Reaktionsbehälter 5, wobei der Rektionsbehälter 5 eine Zuleitung 6 aufweist, über welche eine Suspension 4 in den Reaktionsbehälter 5 einleitbar ist und wobei der Reaktionsbehälter 5 eine Ableitung 7 aufweist, über welche die Suspension 4 aus dem Reaktionsbehälter 5 ausleitbar ist und mit einer Belüftungseinrichtung 8 zur Belüftung der Suspension 4 und/oder eines innerhalb des Reaktionsbehälters 5 angeordneten Trägerelements 10, wobei das Trägerelement 10 wenigstens eine innere und eine äußere Ansiedlungsfläche 11 aufweist, auf welchen sich jeweils ammonifizierende und/oder nitrifizierende Bakterien in einem Biofilm 12 ansiedeln können.

### Bezugszeichenliste

- 1,2,3: Bioreaktor
- 4: Suspension
- 5: Reaktionsbehälter
- 6: Zuleitung
- 7: Ableitung
- 8: Belüftungseinrichtung
- 9: Aufnahmeraum
- 10: Trägerelement
- 11: Ansiedlungsfläche
- 12: Biofilm
- 13: Späne
- 14: Granulat
- 15: Schlauch
- 16: Gaszufuhrleitung
- 17: Kompressor
- 18: Boden des Reaktionsbehälters
- 19: Belüftungsplatte
- 20: Belüftungsöffnungen
- 21: Pumpvorrichtung
- 22: Heizvorrichtung
- 23: Öffnung
- 24: Verschlusseinheit
- 25: Bypassgasleitung
- 26: Hauptgasleitung
- 27: Sammeleinheit
- 28: Weitere Belüftungseinrichtung
- 29: Suspensionskreislauf
- 30: Absperrventil
- 31: Absperrventil
- 32: Bypasszuleitung
- 33: Absperrventil (Luft)
- 34: Absperrventil im Reaktionsbehälter
- 35: Absperrventil im Reaktionsbehälter
- 36: Zerteilereinheit

## Patentansprüche

1. Bioreaktor (1, 2, 3) zur Umwandlung von organischen Rest-und/oder Abfallstoffen in eine organische Nährstofflösung mit einem Anteil von mindestens 10% pflanzenverfügbarem mineralisiertem Stickstoff bezogen auf den Gesamtstickstoffgehalt der Nährstofflösung, mit einem Reaktionsbehälter (5), wobei der Reaktionsbehälter (5) eine Zuleitung (6) aufweist, über welche eine Suspension (4) in den Reaktionsbehälter (5) einleitbar ist, und wobei der Reaktionsbehälter (5) eine Ableitung (7) aufweist, über welche die Suspension (4) aus dem Reaktionsbehälter (5) ausleitbar ist, **dadurch gekennzeichnet, dass** der Bioreaktor (1, 2, 3) eine Belüftungseinrichtung (8) aufweist, mittels welcher Sauerstoff in den Reaktionsbehälter (5) und die darin enthaltene Suspension (4) einleitbar ist, dass in einem Aufnahmeraum (9) des Reaktionsbehälters (5) wenigstens ein Trägerelement (10) mit einer Ansiedlungsfläche (11) zur Ausbildung eines Biofilms (12) aus Mikroorganismen angeordnet ist, wobei das wenigstens eine Trägerelement (10) mit der eingeleiteten Suspension (4) und dem eingeleiteten Sauerstoff umspülbar und/oder durchspülbar ist, und dass ein Oberfläche-zu-Volumen-Verhältnis des Trägerelements (10) größer als ein Oberfläche-zu-Volumen-Verhältnis des Aufnahmeraums (9) ausgestaltet ist.

2. Bioreaktor (1, 2, 3) nach Anspruch 1, **dadurch gekennzeichnet, dass** der Bioreaktor (1, 2, 3) eine Pumpvorrichtung (21) aufweist, insbesondere eine als Kreiselpumpe oder Umwälzpumpe ausgestaltete Pumpvorrichtung (21) aufweist, mittels welcher die Suspension (4) durch die Zuleitung (6) in den Reaktionsbehälter (5) und über die Ableitung (7) aus dem Reaktionsbehälter (5) pumpbar ist, vorzugsweise wobei eine Pumpleistung der Pumpvorrichtung (21) veränderbar ist, und/oder mittels welcher eine Umwälzung der Suspension (4) innerhalb des Reaktionsbehälters (5) möglich ist, vorzugsweise wobei mittels der Pumpvorrichtung (21) eine Suspensionsströmungsrichtung innerhalb des Reaktionsbehälters (5) erzeugbar ist, die wenigstens teilweise entgegen und/oder wenigstens teilweise in einer Sauerstoffströmungsrichtung verläuft, weiter bevorzugt dass die Pumpvorrichtung (21) derart programmierbar ist, dass ein Pumpprogramm mit mehreren Teilschritten automatisiert einstellbar ist.

3. Bioreaktor (1, 2, 3) nach einem der vorstehenden Ansprüche, **dadurch gekennzeichnet, dass** der Bioreaktor (1, 2, 3) mehrere Trägerelemente (10) aufweist, insbesondere welche relativ zueinander beweglich sind, und/oder dass eine Vielzahl von Spänen (13), insbesondere eine Vielzahl von Fließspänen aus Kunststoff, vorgesehen ist, wobei die Späne (13) ungeordnet innerhalb des Reaktionsbehälters (5) angeordnet sind, insbesondere so dass ein Verknäulen der Späne (13) entsteht, vorzugsweise wobei die Späne (13) jeweils eine Länge von 2 cm bis 10 cm und/oder eine Breite von 0,5 cm bis 1,5 cm und/oder eine Dicke von 50 µm bis 500 µm aufweisen.

4. Bioreaktor (1, 2, 3) nach einem der vorstehenden Ansprüche, **dadurch gekennzeichnet, dass** als Trägerelement (10) wenigstens ein poröser Schlauch (15) vorgesehen ist, insbesondere wobei der Schlauch (15) quer oder parallel zu einer Strömungsrichtung des in den Reaktionsbehälter (5) eingeleiteten Sauerstoffs angeordnet ist und/oder quer oder parallel zu einer Strömungsrichtung der in den Reaktionsbehälter (5) eingeleiteten Suspension (4) angeordnet ist, und/oder dass über eine Gaszuführleitung (16) Sauerstoff durch den Schlauch (15) einleitbar ist, insbesondere wobei diese Gaszuführleitung (16) als eine Bypassgasleitung (25) ausgestaltet ist, die von einer Hauptgasleitung (26), vorzugsweise zu einer oder der Belüftungseinrichtung (8) führenden Hauptgasleitung (26), abzweigt.

5. Bioreaktor (1, 2, 3) nach einem der vorstehenden Ansprüche, **dadurch gekennzeichnet, dass** das Trägerelement (10) als Zeolith-Granulat (14), insbesondere mit einer Körnung von 0,6 mm bis 1,0 mm, ausgestaltet ist, vorzugsweise wobei das Zeolith-Granulat (14) in einer innerhalb des Reaktionsbehälters (5) angeordneten Sammeleinheit (27) angeordnet ist und/oder wobei an einem oder dem Boden der Sammeleinheit (27) eine weitere Belüftungseinrichtung (28) angeordnet ist, über welche Sauerstoff in das Zeolith-Granulat (14) einleitbar ist, weiter bevorzugt dass die weitere Belüftungseinrichtung (28) mit einer von einer Hauptgasleitung (26) abgezweigten Bypassgasleitung (25) verbunden ist.

6. Verfahren zur Herstellung einer organischen Nährstofflösung mit einem Anteil von mindestens 10%, insbesondere mindestens 25%, vorzugsweise mindestens 50%, weiter bevorzugt mindestens 75%, pflanzenverfügbarem, mineralisiertem Stickstoff bezogen auf den Gesamtstickstoffgehalt der organischen Nährstofflösung, vorzugsweise wobei ein Nitratanteil am pflanzenverfügbaren, mineralisierten Stickstoff höher als ein Ammoniumanteil ist, umfassend die Schritte:
- in einem Animpfschritt Animpfen eines Trägerelements (10)des Bioreaktors (1, 2, 3) nach einem der vorstehenden Ansprüche mit einem Impfmaterial das ammonifizierende und/oder nitrifizierende Bakterien enthält,
- Ausbilden eines Biofilms (12) mit ammonifizierenden und/oder nitrifizierenden Bakterien auf dem Trägerelement (10),
- in einem Inkubationsschritt Inkubation eines organischen Rest- und/oder Abfallstoffes mit dem Biofilm (12), insbesondere in einem Reaktionsbehälter (5), wobei die ammonifizierenden und/oder die nitrifizierenden Bakterien organisch gebundenen Stickstoff des Rest- und/oder Abfallstoffes in mineralisierten Stickstoff umwandeln,
**dadurch gekennzeichnet, dass** während der Durchführung einzelner oder aller Schritte Sauerstoff in den Reaktionsbehälter (5) und/oder in das Trägerelement (10) eingeleitet wird.

7. Verfahren zur Herstellung eines Substratmaterials zur Kultivierung von Pflanzen, umfassend die Schritte:
- in einem Animpfschritt Animpfen eines Trägerelements (10) des Bioreaktors (1, 2, 3) nach einem der vorstehenden Ansprüche mit einem Impfmaterial das ammonifizierende und/oder nitrifizierende Bakterien enthält,
- Ausbilden eines Biofilms (12) mit ammonifizierenden und/oder nitrifizierenden Bakterien auf dem Trägerelement (10),
**dadurch gekennzeichnet, dass** während der Durchführung einzelner oder aller Schritte Sauerstoff in einen Reaktionsbehälter (5) und/oder in das Trägerelement (10) eingeleitet wird.

8. Verfahren nach einem der vorstehenden Ansprüche, **dadurch gekennzeichnet, dass** ein Anteil an organischem Material des organischen Rest- und/oder Abfallstoffes von 5% bis 60% beträgt und/oder dass ein Kohlenstoff/Stickstoff-Verhältnis des organischen Rest- und/oder Abfallstoffes 11 oder weniger beträgt und/oder das ein Gesamtstickstoffanteil bezogen auf das Gesamtgewicht des organischen Rest- und/oder Abfallstoffes wenigstens 0,3% beträgt und/oder dass ein Anteil an nitratgebundenem Stickstoff bezogen auf den Gesamtgehalt des pflanzenverfügbaren Stickstoffs des organischen Rest- und/oder Abfallstoffes geringer als ein Anteil an ammmoniumgebundenen Stickstoff ist.

9. Verfahren nach einem der vorstehenden Ansprüche, **dadurch gekennzeichnet, dass** der Inkubationsschritt einen Ammonifizierungsschritt und/oder einen Nitrifizierungsschritt aufweist, wobei während des Ammonifizierungsschritts durch die ammonifizierenden Bakterien des Biofilms aus dem organischen Rest- und/oder Abfallstoff organisch gebundener Stickstoff in Ammonium umgewandelt wird und/oder wobei während des Nitrifizierungsschritts durch die nitrifizierenden Bakterien des Biofilms (12) Ammonium in Nitrat umgewandelt wird, insbesondere bis in der organischen Nährlösung mehr Nitrat als Ammonium enthalten ist, vorzugsweise bis ein NO₃:NH₄⁺-Verhältnis von mindestens 2:1, insbesondere von mindestens 3:1, vorzugsweise von mindestens 10:1, vorzugsweise von mindestens 25:1, weiter bevorzugt von mindestens 50:1, vorliegt, weiter bevorzugt wobei der Ammonifizierungsschritt und der Nitrifizierungsschritt in separaten Reaktionsbehältern (5) durchgeführt werden.

10. Verfahren nach einem der vorstehenden Ansprüche, **dadurch gekennzeichnet, dass** als Trägerelement (10) wenigstens ein poröser Schlauch (15), insbesondere ein poröser Gummi-Kunststoff-Schlauch verwendet wird, wobei in den Schlauch (15) zeitlich getrennt Sauerstoff und Impfmaterial und/oder Sauerstoff und Rest- und/oder Abfallstoff eingeleitet werden, vorzugsweise wobei während des Animpfschritts und/oder während des Inkubationsschritts ein Schlauchinnendruck varriert wird, insbesondere indem für einen Zeitraum ein Durchfluss von Impfmaterial und/oder von Rest- und/oder Abfallstoff durch den Schlauch (15) erhöht wird und/oder indem für einen Zeitraum ein Volumenstrom von Sauerstoff durch den Schlauch (15) erhöht wird.

11. Organische Nährstofflösung, insbesondere organischer Pflanzendünger, hergestellt durch das Verfahren nach einem der Ansprüche 6 bis 10, mit einem Anteil von mindestens 25% pflanzenverfügbarem mineralisiertem Stickstoff bezogen auf den Gesamtstickstoffgehalt der organischen Nährstofflösung, wobei ein Nitratanteil am pflanzenverfügbaren, mineralisierten Stickstoff höher als ein Ammoniumanteil ist, wobei der pflanzenverfügbare mineralisierte Stickstoff ausschließlich oder im Wesentlichen aus organisch gebundenem Stickstoff umgesetzt ist, und wobei die organische Nährstofflösung frei von industriell hergestelltem mineralischem Dünger ist.

12. Verwendung eines Substratmaterials zur Kultivierung von Pflanzen, wobei das Substratmaterial einen porösen Schlauch (15) als Trägerelement (10) aufweist, wobei das Substratmaterial als ein Verankerungsmaterial in einem hydroponischen Anbausystemen verwendet wird, wobei die Pflanzen über ihre Wurzeln zumindest teilweise in direktem Kontakt mit einer Außenseite des Trägerelements (10) des Substratmaterials stehen, wobei durch das Trägerelement (10) eine organische Nährstofflösung nach Anspruch 11 geleitet wird, wobei die organische Nährlösung durch Poren in einer Trägerelementwandung von einer Innenseite des Trägerelements (10) zu der Außenseite des Trägerelements (10) diffundiert und/oder gepresst wird, so dass die mit der Außenseite des Trägerelements zumindest teilweise in Kontakt stehenden Wurzeln der Pflanzen den pflanzenverfügbaren, mineralisierten Stickstoff aufnehmen.

13. Verwendung eines Substratmaterials zur Kultivierung von Pflanzen, wobei das Substratmaterial einen porösen Schlauch (15) als Trägerelement (10) aufweist, wobei das Substratmaterial unter eine Pflanzenerde gemischt wird, wobei durch das Trägerelement (10) eine organische Nährstofflösung nach Anspruch 11 geleitet wird, wobei die organische Nährlösung durch Poren in einer Trägerelementwandung von einer Innenseite des Trägerelements (10) zu der Außenseite des Trägerelements (10) diffundiert und/oder gepresst wird.

14. Verwendung eines Substratmaterials zur Kultivierung von Pflanzen, wobei das Substratmaterial einen porösen Schlauch (15) als Trägerelement (10) aufweist, wobei die Pflanzen über ihre Wurzeln zumindest teilweise in direktem Kontakt mit einer Außenseite des Trägerelements (10) des Substratmaterials stehen, und wobei durch das Trägerelement (10) ein organischer Rest- und/oder Abfallstoff geleitet wird, wobei durch die Bakterien des Biofilms (12) organisch gebundener Stickstoff des Rest- und/oder Abfallstoffes in eine organische Nährstofflösung nach Anspruch 11 umwandelt wird, wobei der mineralisierte Stickstoff der Nährstofflösung durch Poren in einer Trägerelementwandung von einer Innenseite des Trägerelements (10) zu der Außenseite des Trägerelements (10) diffundiert und/oder gepresst wird, und wobei die mit der Außenseite des Trägerelements (10) zumindest teilweise in Kontakt stehenden Wurzeln der Pflanzen den pflanzenverfügbaren, mineralisierten Stickstoff aufnehmen.

15. Set aus einem Bioreaktor nach einem der Ansprüche 1 bis 5 und einem Impfmaterial zum Animpfen des Trägerelements (10) und zur Ausbildung eines Biofilms (12) mit ammonifizierenden und/oder nitrifizierenden Bakterien.

## Claims

1. Bioreactor (1, 2, 3) for conversion of organic residual and/or waste materials into an organic nutrient solution having a proportion of at least 10% plant-available mineralized nitrogen based on the total nitrogen content of the nutrient solution, comprising a reaction tank (5), wherein the reaction tank (5) has a supply line (6) via which a suspension (4) is introducible into the reaction tank (5), and wherein the reaction tank (5) has a discharge line (7) via which the suspension (4) is dischargeable from the reaction tank (5), **characterized in that** the bioreactor (1, 2, 3) has an aeration system (8) by means of which oxygen is introducible into the reaction tank (5) and the suspension (4) contained therein, **in that** there is arranged in an accommodation space (9) of the reaction tank (5) at least one support element (10) having a colonization surface (11) for formation of a biofilm (12) of microorganisms, wherein the introduced suspension (4) and the introduced oxygen are flushable around and/or through the at least one support element (10), and **in that** a surface-to-volume ratio of the support element (10) is configured to be greater than a surface-to-volume ratio of the accommodation space (9).

2. Bioreactor (1, 2, 3) according to Claim 1, **characterized in that** the bioreactor (1, 2, 3) has a pump device (21), especially a pump device (21) configured as a centrifugal pump or circulating pump, by means of which the suspension (4) is pumpable into the reaction tank (5) through the supply line (6) and out of the reaction tank (5) via the discharge line (7), preferably wherein a pump performance of the pump device (21) is alterable, and/or by means of which a circulation of the suspension (4) within the reaction tank (5) is possible, preferably wherein a suspension flow direction is generatable within the reaction tank (5) by means of the pump device (21), which suspension flow direction runs at least partially against and/or at least partially in an oxygen flow direction, further preferably **in that** the pump device (21) is programmable such that a pumping program comprising multiple sub-steps is automatically settable.

3. Bioreactor (1, 2, 3) according to either of the preceding claims, **characterized in that** the bioreactor (1, 2, 3) has multiple support elements (10), especially which are movable relative to one another, and/or **in that** a multiplicity of chips (13), especially a multiplicity of flow chips made of plastic, is provided, wherein the chips (13) are arranged within the reaction tank (5) in a disordered manner, especially such that an entanglement of the chips (13) arises, preferably wherein the chips (13) each have a length of 2 cm to 10 cm and/or a width of 0.5 cm to 1.5 cm and/or a thickness of 50 µm to 500 µm.

4. Bioreactor (1, 2, 3) according to any of the preceding claims, **characterized in that** at least one porous hose (15) is provided as support element (10), especially wherein the hose (15) is arranged transversely or parallel to a flow direction of the oxygen introduced into the reaction tank (5) and/or is arranged transversely or parallel to a flow direction of the suspension (4) introduced into the reaction tank (5), and/or **in that** oxygen is introducible through the hose (15) via a gas feed line (16), especially wherein said gas feed line (16) is configured as a bypass gas line (25) which branches off from a main gas line (26), preferably main gas line (26) leading towards an aeration system (8) or the aeration system (8).

5. Bioreactor (1, 2, 3) according to any of the preceding claims, **characterized in that** the support element (10) is configured as zeolite granular material (14), especially with a grain size of 0.6 mm to 1.0 mm, preferably wherein the zeolite granular material (14) is arranged in a collection unit (27) arranged within the reaction tank (5) and/or wherein there is arranged on a base or the base of the collection unit (27) a further aeration system (28) via which oxygen is introducible into the zeolite granular material (14), further preferably **in that** the further aeration system (28) is connected to a bypass gas line (25) which branches off from a main gas line (26).

6. Method for producing an organic nutrient solution having a proportion of at least 10%, especially at least 25%, preferably at least 50%, further preferably at least 75%, plant-available, mineralized nitrogen based on the total nitrogen content of the organic nutrient solution, preferably wherein a proportion of nitrate in the plant-available, mineralized nitrogen is higher than a proportion of ammonium, comprising the steps of:
- inoculating, in an inoculation step, a support element (10) of the bioreactor (1, 2, 3) according to any of the preceding claims with an inoculation material containing ammonifying and/or nitrifying bacteria,
- forming a biofilm (12) containing ammonifying and/or nitrifying bacteria on the support element (10),
- incubating, in an incubation step, an organic residual and/or waste material with the biofilm (12), especially in a reaction tank (5), wherein the ammonifying and/or the nitrifying bacteria convert organically bound nitrogen of the residual and/or waste material into mineralized nitrogen,
**characterized in that** oxygen is introduced into the reaction tank (5) and/or into the support element (10) while individual steps or all the steps are being carried out.

7. Method for producing a substrate material for cultivation of plants, comprising the steps of:
- inoculating, in an inoculation step, a support element (10) of the bioreactor (1, 2, 3) according to any of the preceding claims with an inoculation material containing ammonifying and/or nitrifying bacteria,
- forming a biofilm (12) containing ammonifying and/or nitrifying bacteria on the support element (10),
**characterized in that** oxygen is introduced into a reaction tank (5) and/or into the support element (10) while individual steps or all the steps are being carried out.

8. Method according to any of the preceding claims, **characterized in that** a proportion of organic material of the organic residual and/or waste material is from 5% to 60% and/or **in that** a carbon/nitrogen ratio of the organic residual and/or waste material is 11 or less and/or **in that** a total nitrogen proportion based on the total weight of the organic residual and/or waste material is at least 0.3% and/or **in that** a proportion of nitrate-bound nitrogen based on the total content of the plant-available nitrogen of the organic residual and/or waste material is less than a proportion of ammonium-bound nitrogen.

9. Method according to any of the preceding claims, **characterized in that** the incubation step comprises an ammonification step and/or a nitrification step, wherein organically bound nitrogen is converted into ammonium from the organic residual and/or waste material by the ammonifying bacteria of the biofilm during the ammonification step and/or wherein ammonium is converted into nitrate by the nitrifying bacteria of the biofilm (12) during the nitrification step, especially until more nitrate than ammonium is contained in the organic nutrient solution, preferably until there is an NO₃:NH₄⁺ ratio of at least 2:1, especially of at least 3:1, preferably of at least 10:1, preferably of at least 25:1, further preferably of at least 50:1, further preferably wherein the ammonification step and the nitrification step are carried out in separate reaction tanks (5).

10. Method according to any of the preceding claims, **characterized in that** at least one porous hose (15), especially a porous rubber-plastic hose, is used as support element (10), wherein oxygen and inoculation material and/or oxygen and residual and/or waste material are introduced into the hose (15) at separate times, preferably wherein an internal hose pressure is varied during the inoculation step and/or during the incubation step, especially by increasing a flow of inoculation material and/or of residual and/or waste material through the hose (15) for a period of time and/or by increasing a volume flow of oxygen through the hose (15) for a period of time.

11. Organic nutrient solution, especially organic plant fertilizer, produced by the method according to any of Claims 6 to 10, having a proportion of at least 25% plant-available mineralized nitrogen based on the total nitrogen content of the organic nutrient solution, wherein a proportion of nitrate in the plant-available, mineralized nitrogen is higher than a proportion of ammonium, wherein the plant-available mineralized nitrogen has been converted exclusively or substantially from organically bound nitrogen, and wherein the organic nutrient solution is free of industrially produced mineral fertilizer.

12. Use of a substrate material for cultivation of plants, wherein the substrate material has a porous hose (15) as support element (10), wherein the substrate material is used as an anchoring material in a hydroponic cultivation system, wherein the plants are, via their roots, at least partially in direct contact with an outer side of the support element (10) of the substrate material, wherein an organic nutrient solution according to Claim 11 is conducted through the support element (10), wherein the organic nutrient solution diffuses and/or is pressed through pores in a support-element wall from an inner side of the support element (10) towards the outer side of the support element (10), with the result that the roots of the plants that are at least partially in contact with the outer side of the support element take up the plant-available, mineralized nitrogen.

13. Use of a substrate material for cultivation of plants, wherein the substrate material has a porous hose (15) as support element (10), wherein the substrate material is mingled with a plant soil, wherein an organic nutrient solution according to Claim 11 is conducted through the support element (10), wherein the organic nutrient solution diffuses and/or is pressed through pores in a support-element wall from an inner side of the support element (10) towards the outer side of the support element (10).

14. Use of a substrate material for cultivation of plants, wherein the substrate material has a porous hose (15) as support element (10), wherein the plants are, via their roots, at least partially in direct contact with an outer side of the support element (10) of the substrate material, and wherein an organic residual and/or waste material is conducted through the support element (10), wherein organically bound nitrogen of the residual and/or waste material is converted into an organic nutrient solution according to Claim 11 by the bacteria of the biofilm (12), wherein the mineralized nitrogen of the nutrient solution diffuses and/or is pressed through pores in a support-element wall from an inner side of the support element (10) towards the outer side of the support element (10), and wherein the roots of the plants that are at least partially in contact with the outer side of the support element (10) take up the plant-available, mineralized nitrogen.

15. Set composed of a bioreactor according to any of Claims 1 to 5 and an inoculation material for inoculation of the support element (10) and for formation of a biofilm (12) containing ammonifying and/or nitrifying bacteria.

## Revendications

1. Bioréacteur (1, 2, 3) pour transformer des résidus et/ou des déchets organiques en une solution nutritive organique avec une proportion d'au moins 10% d'azote minéralisé disponible pour les végétaux par rapport à la teneur totale en azote de la solution nutritive, avec une cuve de réaction (5), dans lequel la cuve de réaction (5) présente une conduite d'alimentation (6) par laquelle une suspension (4) peut être introduite dans la cuve de réaction (5), et dans lequel la cuve de réaction (5) présente une conduite d'évacuation (7) par laquelle la suspension (4) peut être évacuée de la cuve de réaction (5), **caractérisé en ce que** ce bioréacteur (1, 2, 3) présente un dispositif de ventilation (8) au moyen duquel de l'oxygène peut être introduit dans la cuve de réaction (5) et dans la suspension (4) qu'elle contient, que dans un espace de réception (9) de la cuve de réaction (5) est disposé au moins un élément de support (10) avec une surface d'implantation (11) pour la formation d'un biofilm (12) de micro-organismes, dans lequel l'au moins un élément de support (10) peut être baigné et/ou arrosé avec la suspension (4) introduite et l'oxygène introduit, et qu'un rapport surface/volume de l'élément de support (10) supérieur à un rapport surface/volume de l'espace de réception (9) est créé.

2. Bioréacteur (1, 2, 3) selon la revendication 1, **caractérisé en ce que** ce bio réacteur (1, 2, 3) présente un dispositif de pompe (21), en particulier présente un dispositif de pompage (21) configuré sous la forme d'une pompe centrifuge ou d'une pompe de circulation, au moyen duquel la suspension (4) peut être pompée à travers la conduite d'alimentation (6) dans la cuve de réaction (5) et par la conduite d'évacuation (7) hors de la cuve de réaction, une puissance de pompe du dispositif de pompage (21) étant de préférence modifiable, et/ou au moyen duquel une circulation de la suspension (4) à l'intérieur de la cuve de réaction (5) est possible, un sens de circulation de la suspension à l'intérieur de la cuve de réaction (5) qui est au moins partiellement opposée et/ou au moins partiellement identique au sens de l'écoulement de l'oxygène pouvant préférence être créé au moyen du dispositif de pompage (21), le dispositif de pompage (21) étant plus préférablement programmable de sorte qu'un programme de pompage avec plusieurs étapes partielles peut être configuré automatiquement.

3. Bioréacteur (1, 2, 3) selon une des revendications précédentes, **caractérisé en ce que** ce bioréacteur (1, 2, 3) présente plusieurs éléments de support (10), lesquels sont en particuliers mobiles les une par rapport aux autres, et/ou qu'une multitude de copeaux (13), en particulier une multitude de copeaux circulants en plastique, sont prévus, les copeaux (13) étant disposés de façon désordonnée à l'intérieur de la cuve de réaction (5), en particulier de sorte qu'un enchevêtrement des copeaux (13) se produit, les copeaux (13) présentant de préférence chacun une longueur de 2 cm à 10 cm et/ou une largeur de 0,5 cm à 1,5 cm et/ou une épaisseur de 50 µm à 500 µm.

4. Bioréacteur (1, 2, 3) selon une des revendications précédentes, **caractérisé en ce qu'**en guise d'élément de support (10), il est prévu au moins un tuyau poreux (15), le tuyau (15) étant en particulier disposé perpendiculairement ou parallèlement à un sens d'écoulement de l'oxygène introduit dans la cuve de réaction (5) et/ou disposé perpendiculairement ou parallèlement à un sens d'écoulement de la suspension (4) introduite dans la cuve de réaction (5), et/ou que de l'oxygène peut être introduit par une conduite d'alimentation en gaz (16) à travers le tuyau (15), cette conduite d'alimentation en gaz (17) étant en particulier conçue comme une conduite de dérivation de gaz (25) qui bifurque à partir d'une conduite de gaz principale (26), de préférence une conduite de gaz principale (26) menant vers un ou le dispositif de ventilation (8).

5. Bioréacteur (1, 2, 3) selon une des revendications précédentes, **caractérisé en ce que** l'élément de support (10) est conçu comme un granulat de zéolithe (14), en particulier d'une granulosité de 0,6 mm à 1,0 mm, le granulat de zéolithe (14) étant de préférence disposé dans un collecteur (27) disposé à l'intérieur de la cuve de réaction (5) et/ou sur un ou le fond du collecteur du collecteur (27) étant disposé un autre dispositif de ventilation (28) par le biais duquel de l'oxygène peut être introduit dans le granulat de zéolithe (14), et que plus préférablement l'autre dispositif de ventilation (28) est relié avec une conduite de dérivation de gaz (25) bifurquant à partir d'une conduite de gaz principale (26).

6. Procédé pour fabriquer une solution nutritive organique avec une proportion d'au moins 10%, en particulier d'au moins 25%, de préférence d'au moins 50%, plus préférablement d'au moins 75% d'azote minéralisé disponible pour les végétaux par rapport à la teneur totale en azote de la solution nutritive, selon lequel une proportion de nitrate dans l'azote minéralisé disponible pour les végétaux est de préférence supérieure à une proportion d'ammonium, comprenant les étapes suivantes :
- dans une étape d'ensemencement, ensemencement d'un élément de support (10) du bioréacteur (1, 2, 3) selon une des revendications précédentes avec une matière d'ensemencement qui contient des bactéries ammonifiantes et/ou nitrifiantes,
- formation d'un biofilm (12) avec des bactéries ammonifiantes et/ou nitrifiantes sur l'élément de support (10),
- dans une étape d'incubation, incubation de résidus et/ou de déchets organiques avec le biofilm (12), en particulier dans une cuve de réaction (5), dans laquelle les bactéries ammonifiantes et/ou nitrifiantes transforment l'azote relié organiquement des résidus et/ou des déchets en azote minéralisé,
**caractérisé en ce que**, pendant l'exécution de certaines ou de toutes les étapes, de l'oxygène est introduit dans la cuve de réaction (5) et/ou dans l'élément de support (10).

7. Procédé pour fabriquer une matière de substrat pour la culture des végétaux, comprenant les étapes suivantes :
- dans une étape d'ensemencement, ensemencement d'un élément de support (10) du bioréacteur (1, 2, 3) selon une des revendications précédentes avec une matière d'ensemencement qui contient des bactéries ammonifiantes et/ou nitrifiantes,
- formation d'un biofilm (12) avec des bactéries ammonifiantes et/ou nitrifiantes sur l'élément de support (10),
**caractérisé en ce que**, pendant l'exécution de certaines ou de toutes les étapes, de l'oxygène est introduit dans une cuve de réaction (5) et/ou dans l'élément de support (10).

8. Procédé selon une des revendications précédentes, **caractérisé en ce qu'**une proportion de matière organique des résidus et/ou des déchets organiques est de 5% à 60% et/ou qu'un rapport carbone/azote des résidus et/ou des déchets organiques est de 11 ou moins et/ou qu'une proportion d'azote totale par rapport au poids total des résidus et/ou des déchets organiques est d'au moins 0,3% et/ou qu'une proportion d'azote lié au nitrate par rapport à la teneur totale en azote disponible pour les végétaux des résidus et/ou des déchets organiques est inférieure à une proportion d'azote lié à l'ammonium.

9. Procédé selon une des revendications précédentes, **caractérisé en ce que** l'étape d'incubation comprend une étape d'ammonification et/ou une étape de nitrification, dans lequel l'azote lié organiquement est transformé en ammonium par les bactéries ammonifiantes du biofilm provenant des résidus et/ou des déchets organiques au cours de l'étape d'ammonification et/ou l'ammonium est transformé en nitrate par les bactéries nitrifiantes du biofilm (12) au cours de l'étape de nitrification, en particulier jusqu'à ce que davantage de nitrate que d'ammonium soit contenu dans la solution nutritive organique, de préférence jusqu'à ce qu'un rapport NO₃:NH₄⁺ d'au moins 2:1, en particulier d'au moins 3:1, de préférence d'au moins 10:1, de préférence d'au moins 25:1, plus préférablement d'au moins 50:1 soit obtenu, l'étape d'ammonification et l'étape de nitrification étant en outre effectuées de préférence dans des cuves de réaction (5) séparées.

10. Procédé selon une des revendications précédentes, **caractérisé en ce qu'**en guide d'élément de support (10) est utilisé au moins un tuyau poreux (15), en particulier un tuyau en caoutchouc-plastique poreux, l'oxygène et la matière d'ensemencement et/ou l'oxygène et les résidus et/ou déchets étant introduits dans le tuyau (15) à des moments différents, une pression intérieure du tuyau variant de préférence pendant l'étape d'ensemencement et/ou pendant l'étape d'incubation, en particulier du fait que, pendant un laps de temps, un débit de matière d'ensemencement et/ou de résidus et/ou de déchets à travers le tuyau (15) est augmenté et/ou du fait que, pendant un laps de temps, un débit volumique d'oxygène à travers le tuyau (15) est augmenté.

11. Solution nutritive organique, en particulier engrais organique, fabriqué par le procédé selon une des revendications 6 à 10, avec une proportion d'au moins 25% d'azote minéralisé disponible pour les végétaux par rapport à la teneur totale en azote de la solution nutritive organique, une proportion de nitrate dans l'azote minéralisé disponible pour les végétaux étant supérieure à une proportion d'ammonium, l'azote minéralisé disponible pour les végétaux étant exclusivement ou essentiellement transformé en azote lié organiquement, et cette solution nutritive organique étant exempte d'engrais minéral fabriqué industriellement.

12. Utilisation d'une matière de substrat pour la culture des végétaux, dans laquelle la matière de substrat possède un tuyau poreux (15) comme élément de support (10), dans laquelle la matière de substrat est utilisée comme matière d'ancrage dans un système de culture hydroponique, dans laquelle les végétaux sont au moins partiellement en contact direct par leurs racines avec une face externe de l'élément de support (10) de la matière de substrat, dans laquelle une solution nutritive organique selon la revendication 11 est conduite à travers l'élément de support (10), dans laquelle la solution nutritive organique est diffusée et/ou pressée à travers des pores dans une paroi de l'élément de support d'une face interne de l'élément de support (10) jusqu'à une face externe de l'élément de support (10), de sorte que les racines des végétaux au moins partiellement en contact avec la face externe de l'élément de support absorbent l'azote minéralisé disponible pour les végétaux.

13. Utilisation d'une matière de substrat pour la culture des végétaux, dans laquelle la matière de substrat possède un tuyau poreux (15) comme élément de support (10), dans laquelle la matière de substrat est mélangée sous un terreau, dans laquelle une solution nutritive organique selon la revendication 11 est conduite à travers l'élément de support (10), dans laquelle la solution nutritive organique est diffusée et/ou pressée à travers des pores dans une paroi de l'élément de support d'une face interne de l'élément de support (10) jusqu'à une face externe de l'élément de support (10).

14. Utilisation d'une matière de substrat pour la culture des végétaux, dans laquelle la matière de substrat possède un tuyau poreux (15) comme élément de support (10), dans laquelle les végétaux sont au moins partiellement en contact direct par leurs racines avec une face externe de l'élément de support (10) de la matière de substrat et dans laquelle des résidus et/ou des déchets organiques sont conduits à travers l'élément de support (10), dans laquelle l'azote lié organiquement des résidus et/ou des déchets est transformé en une solution nutritive organique selon la revendication 11 par les bactéries du biofilm (12), dans laquelle l'azote minéralisé de la solution nutritive est diffusé et/ou pressé à travers des pores dans une paroi de l'élément de support d'une face interne de l'élément de support (10) jusqu'à une face externe de l'élément de support (10), et dans laquelle les racines des végétaux au moins partiellement en contact avec la face externe de l'élément de support (10) absorbent l'azote minéralisé disponible pour les végétaux.

15. Ensemble constitué d'un bioréacteur selon une des revendications 1 à 5 et d'une matière d'ensemencement pour ensemencer l'élément de support (10) et pour former un biofilm (12) avec des bactéries ammonifiantes et/ou nitrifiantes.
